# EUROPEAN PATENT SPECIFICATION

(11) **EP 2 411 440 B1**
(45) Date of publication and mention of the grant of the patent: **17.01.2018**
(21) Application number: 10756701.8
(22) Date of filing: 23.03.2010
(51) Int. Cl.: C08G 63/08, C08G 63/90, C08L 67/04, C09D 167/04, C08J 7/04

(54) **IMPROVED BIODEGRADABLE POLYMERS**
VERBESSERTE BIOLOGISCH ABBAUBARE POLYMERE
POLYMÈRES BIODÉGRADABLES AMÉLIORÉS

(30) Priority: 23.03.2009 US 162653 P
(43) Date of publication of application: 01.02.2012
(73) Proprietor: Micell Technologies, Inc., Durham, NC 27713-4384 (US)
(72) Inventor: TAYLOR, Douglas, Franklinton NC 27525 (US); MCCLAIN, James, B., Raleigh NC 27613 (US); SCHMITT, Edward, Palo Alto CA 94306 (US)
(74) Representative: Wichmann, Hendrik
(86) International application number: PCT/US2010/028265
(87) International publication number: WO 2010/111238

(56) References cited:
- EP-A2- 0 202 065
- EP-A2- 0 283 925
- EP-A2- 0 452 111
- EP-A2- 0 469 520
- WO-A1-84/01508
- WO-A1-2006/118808
- WO-A1-2008/131131
- WO-A1-2008/131131
- WO-A1-2009/016194
- WO-A1-2010/053242
- WO-A2-2005/117942
- WO-A2-2006/099276
- US-A- 5 811 032
- US-A1- 2007 179 276
- US-A1- 2008 269 449

## Description

### CROSS REFERENCE

### SUMMARY OF THE INVENTION

Subject-matter of the present invention are methods according to claims 1-4 as attached.

The present invention relates to compositions comprising biodegradable polymers with improved properties and methods for the preparation of said biodegradable polymers. The present invention also relates to substrates coated with these compositions and a pharmaceutical or biological agent in powder form, and to methods for depositing these coating compositions and a pharmaceutical or biological agent in powder form onto a substrate. Additionally, the present invention relates to bioabsorable biomedical devices made from such polymers, including sutures, wound closure devices, and orthopedic devices such as screws, pins and plates. Further, the present invention relates to drug-delivery matricies and depots. As well, the invention relates to devices having coatings comprising the polymers provided herein such as coated stents, including coronary and peripheral stents; coated balloons and other medical devices as described below.

Biodegradable polymers are employed in a variety of applications, such as drug delivery, medical devices and implantable structural devices.

For example, it is useful to coat biomedical implants to provide for the localized delivery of pharmaceutical or biological agents to target specific locations within the body, for therapeutic or prophylactic benefit. One area of particular interest is drug eluting stents (DES) that has recently been reviewed by Ong and Serruys in Nat. Clin. Pract. Cardiovasc. Med., (Dec 2005), Vol 2, No 12, 647. Typically such pharmaceutical or biological agents are co-deposited with a polymer. Such localized delivery of these agents avoids the problems of systemic administration, which may be accompanied by unwanted effects on other parts of the body, or because administration to the afflicted body part requires a high concentration of pharmaceutical or biological agent that may not be achievable by systemic administration. The coating may provide for controlled release, including long-term or sustained release, of a pharmaceutical or biological agent. Additionally, biomedical implants may be coated with materials to provide beneficial surface properties, such as enhanced biocompatibility or lubriciousness.

Poly(alpha-hydroxycarboxylic acids) include polymers of lactic acid, polymers of glycolic acid, and co-polymers of lactic acid and glycolic acid (PLGA). Poly(alpha-hydroxycarboxylic acids), are a group of copolymers approved for numerous therapeutic uses owing to its biodegradability and biocompatibility. PLGA is prepared by the ring-opening polymerization of the cyclic dimers (1,4-dioxane-2,5-dione) of glycolic acid and lactic acid. Catalysts used to initatiate the ring-open polymerization include tin(II) alkoxides or aluminium alkoxides. PLGA has good solubility in many organic solvents. Different forms of PLGA, with varying rates of hydrolysis, are produced by varying the ratio of glycolic acid monomer to lactic acid monomer. In addition, co-polymers in which the carboxy terminus is capped with an alkyl group have enhanced stability. Provided herein is a composition comprising a poly(alpha-hydroxycarboxylic acid) substantially free of acidic impurities.

In one embodiment is a composition comprising poly(D,L-lactic-co-glycolic acid) substantially free of acidic impurities.

In another embodiment is a composition comprising poly(L-lactic acid) substantially free of acidic impurities.

In another embodiment is a composition comprising poly(D-lactic acid) substantially free of acidic impurities.

In another embodiment is a composition comprising poly(D,L-lactic acid) substantially free of acidic impurities.

In some embodiments, the poly(D,L-lactic-co-glycolic acid) contains less than 0.5 % (wt/wt) of acidic impurity. In some embodiments, the poly(D,L-lactic-co-glycolic acid) contains less than 1.0 % (wt/wt) of acidic impurity. In some embodiments, the poly(D,L-lactic-co-glycolic acid) contains less than 1.5 % (wt/wt) of acidic impurity. In some embodiments, the poly(D,L-lactic-co-glycolic acid) has a ratio of lactic acid monomer to glycolic acid monomer ranging from 82:18 to 88:12. In some embodiments, the poly(D,L-lactic-co-glycolic acid) has a ratio of lactic acid monomer to glycolic acid monomer ranging from 72:28 to 78:22. In some embodiments, the poly(D,L-lactic-co-glycolic acid) has a ratio of lactic acid monomer to glycolic acid monomer ranging from 62:38 to 68:32. In some embodiments, the poly(D,L-lactic-co-glycolic acid) has a ratio of lactic acid monomer to glycolic acid monomer ranging from 47:53 to 53:47.

In some embodiments, the poly(D,L-lactic-co-glycolic acid) has a weight average molecular weight of about 4,000 to about 8,000. In some embodiments, the poly(D,L-lactic-co-glycolic acid) has a weight average molecular weight of about 8,000 to about 12,000. In some embodiments, the poly(D,L-lactic-co-glycolic acid) has a weight average molecular weight of about 12,000 to about 16,000. In some embodiments, the poly(D,L-lactic-co-glycolic acid) has a weight average molecular weight of up to about 50 kDalton. In some embodiments, the poly(D,L-lactic-co-glycolic acid) has a weight average molecular weight of up to about 90 kDalton. In some embodiments, the poly(D,L-lactic-co-glycolic acid) has a weight average molecular weight of up to about 120 kDalton.

Provided herein is a method for the preparation of a composition comprising poly(D,L-lactic-co-glycolic acid) substantially free of acidic impurities, said method comprising: contacting poly(D,L-lactic-co-glycolic acid) containing acidic impurties with a solid base; forming a salt of the acidic impurity; and separating the poly(D,L-lactic-co-glycolic acid) from the salt of the acidic impurity.

In some embodiments, the solid base is selected from the group consisting of: MgO, LiH, NaH, KH, MgH₂, and CaH₂. In some embodiments, the solid base is CaH₂.

Provided herein is a method for the preparation of a composition comprising poly(D,L-lactic-co-glycolic acid) substantially free of acidic impurities, said method comprising: dissolving the poly(D,L-lactic-co-glycolic acid) containing acidic impurties in an inert solvent; contacting poly(D,L-lactic-co-glycolic acid) solution with a metal hydride; forming a metal salt of the acidic impurity; and separating the poly(D,L-lactic-co-glycolic acid) from the metal salt of the acidic impurity.

In some embodiments, the solid base is selected from the group consisting of: MgO, LiH, LiAlH₄, NaH, NaBH₄, KH, MgH₂, and CaH₂. In some embodiments, the solid base is CaH₂. In some embodiments, the metal salt of the acidic impurity is separated from the poly(D,L-lactic-co-glycolic acid) by filtration. In some embodiments, the inert solvent is an organic solvent. In some embodiments, the salt of the acidic impurity is separated from the poly(D,L-lactic-co-glycolic acid) by diffusion through a semi-permeable membrane.

In some embodiments, the method is performed in a supercritical state.

In some embodiments, the inert solvent is a fluorocarbon. In some embodiments, the fluorocarbon solvent is FC236.

Provided herein is a method for the preparation of a composition comprising poly(D,L-lactic-co-glycolic acid) substantially free of acidic impurities, said method comprising: forming the poly(D,L-lactic-co-glycolic acid) containing acidic impurties into a thin film; contacting said poly(D,L-lactic-co-glycolic acid) thin film with a layer of solid base; diffusing the acidic impurties from said poly(D,L-lactic-co-glycolic acid) thin film; and separating the poly(D,L-lactic-co-glycolic acid) thin film from the layer of solid base.

In some embodiments, the solid base is selected from the group consisting of: MgO, LiH, NaH, KH, MgH₂, and CaH₂. In some embodiments, the solid base is CaH₂.

Provided herein is a method for the preparation of a composition comprising poly(D,L-lactic-co-glycolic acid) substantially free of acidic impurities, said method comprising subjecting the poly(D,L-lactic-co-glycolic acid) containing acidic impurties to electrophoresis.

Provided herein is a device comprising: a substrate, and a coating wherein the coating comprises poly(D,L-lactic-co-glycolic acid) substantially free of acidic impurities.

Provided herein is a device comprising: a substrate, and a coating wherein the coating comprises the composition comprising poly(D,L-lactic-co-glycolic acid) substantially free of acidic impurities.

Provided herein is a device comprising: a substrate, and a coating wherein the coating comprises the composition comprising poly(D,L-lactic-co-glycolic acid) substantially free of acidic impurities, formed by any of the methods described herein.

In some embodiments, the substrate comprises a stent framework. In some embodiments, the substrate is a biomedical implant selected from the group consisting of stents (e.g., vascular stents), electrodes, catheters, leads, implantable pacemaker, cardioverter or defibrillator housings, joints, screws, rods, ophthalmic implants, femoral pins, bone plates, grafts, anastomotic devices, perivascular wraps, sutures, staples, shunts for hydrocephalus, dialysis grafts, colostomy bag attachment devices, ear drainage tubes, leads for pace makers and implantable cardioverters and defibrillators, vertebral disks, bone pins, suture anchors, hemostatic barriers, clamps, screws, plates, clips, vascular implants, tissue adhesives and sealants, tissue scaffolds, various types of dressings (e.g., wound dressings), bone substitutes, intraluminal devices, and vascular supports.

In some embodiments, the coating comprises rapamycin wherein at least part of rapamycin is in crystalline form.

In some embodiments, the coating has substantially uniform thickness and rapamycin in the coating is substantially uniformly dispersed.

In some embodiments, the average rapamycin content varies along the length of said device.

In some embodiments, at least part of said rapamycin forms a phase separate from one or more phases formed by said poly(D,L-lactic-co-glycolic acid) .

In some embodiments, the rapamycin is at least 50% crystalline. In some embodiments, the rapamycin is at least 75% crystalline. In some embodiments, the rapamycin is at least 90% crystalline. In some embodiments, the rapamycin is at least 95% crystalline. In some embodiments, the rapamycin is at least 99% crystalline.

In some embodiments, the polymer is a mixture of two or more polymers, wherein at least one of the polymers is said poly(D,L-lactic-co-glycolic acid). In some embodiments, the mixture of polymers forms a continuous film around particles of rapamycin. In some embodiments, two or more polymers are intimately mixed. In some embodiments, the mixture comprises no single polymer domain larger than about 20 nm. In some embodiments, each polymer in said mixture comprises a discrete phase. In some embodiments, the discrete phases formed by said polymers in said mixture are larger than about 10nm. In some embodiments, the discrete phases formed by said polymers in said mixture are larger than about 50nm.

In some embodiments, the rapamycin in said device has a shelf stability of at least 3 months. In some embodiments, the rapamycin in said device has a shelf stability of at least 6 months. In some embodiments, the rapamycin in said device has a shelf stability of at least 12 months. In some embodiments, the device provides an elution profile wherein about 10% to about 50% of rapamycin is eluted at week 1 after the composite is implanted in a subject under physiological conditions, about 25% to about 75% of rapamycin is eluted at week 2 and about 50% to about 100% of rapamycin is eluted at week 4.

In some embodiments, the coating comprises a macrolide immunosuppressive (limus) drug-polymer coating wherein at least part of the drug is in crystalline form. In some embodiments, the macrolide immunosuppressive drug comprises one or more of rapamycin, biolimus (biolimus A9), 40-0-(2-Hydroxyethyl)rapamycin (everolimus), 40-0-Benzyl-rapamycin, 40-0-(4'-Hydroxymethyl)benzyl-rapamycin, 40-O-[4'-(1,2-Dihydroxyethyl)]benzyl-rapamycin, 40-0-Allyl-rapamycin, 40-O-[3'-(2,2-Dimethyl1-1,3-dioxolan-4(S)-yl)-prop-2'-en-1'-yl]-rapamycin, (2':E,4'S)-40-O-(4',5'-Dihydroxypent-2'-en-1'-yl)-rapamycin 40-O-(2-Hydroxy)ethoxycar-bonylmethyl-rapamycin, 40-O-(3-Hydroxy)propyl-rapamycin 4O-O-(6-Hydroxy)hexyl-rapamycin 40-O-[2-(2-Hydroxy)ethoxy]ethyl-rapamycin 4O-O-[(3S)-2,2-Dimethyldioxolan-3-yl]methyl-rapamycin, 40-O-[(2S)-2,3-Dihydroxyprop-1-yl]-rapamycin, 4O-O-(2-Acetoxy)ethyl-rapamycin 4O-O-(2-Nicotinoyloxy)ethyl-rapamycin, 4O-O-[2-(N-Morpholino)acetoxy]ethyl-rapamycin 4O-O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methyl-rapamycin, 4O-O-(2-Aminoethyl)-rapamycin, 4O-O-(2-Acetaminoethyl)-rapamycin 4O-O-(2-Nicotinamidoethyl)-rapamycin, 4O-O-(2-(N-Methyl-imidazo-2'-ylcarbethoxamido)ethyl)-rapamycin, 4O-O-(2-Ethoxycarbonylaminoethyl)-rapamycin, 40-0-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), and 42-[3-hydroxy-2-(hydroxymethyl)-2-methylpropanoate]rapamycin (temsirolimus), (42S)-42-Deoxy-42-(1H-tetrazol-1-yl)-rapamycin (zotarolimus), and salts, derivatives, isomers, racemates, diastereoisomers, prodrugs, hydrate, ester, or analogs thereof. In some embodiments, the macrolide immunosuppressive drug is at least 50% crystalline.

In some embodiments, the coating comprises a pharmaceutical agent. In some embodiments, the pharmaceutical agent is selected form the group consisting of antirestenotic agents, antidiabetics, analgesics, antiinflammatory agents, antirheumatics, antihypotensive agents, antihypertensive agents, psychoactive drugs, tranquillizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals, chemotherapeutic agents and amino acids. Examples of suitable active ingredients are acarbose, acetylsalicylic acid, acyclovir, allopurinol, alprostadil, prostaglandins, amantadine, ambroxol, amlodipine, S-aminosalicylic acid, amitriptyline, atenolol, azathioprine, balsalazide, beclomethasone, betahistine, bezafibrate, diazepam and diazepam derivatives, budesonide, bufexamac, buprenorphine, methadone, calcium salts, potassium salts, magnesium salts, candesartan, carbamazepine, captopril, cetirizine, chenodeoxycholic acid, theophylline and theophylline derivatives, trypsins, cimetidine, clobutinol, clonidine, cotrimoxazole, codeine, caffeine, vitamin D and derivatives of vitamin D, colestyramine, cromoglicic acid, coumarin and coumarin derivatives, cysteine, ciclosporin, cyproterone, cytabarine, dapiprazole, desogestrel, desonide, dihydralazine, diltiazem, ergot alkaloids, dimenhydrinate, dimethyl sulphoxide, dimeticone, domperidone and domperidan derivatives, dopamine, doxazosin, doxylamine, benzodiazepines, diclofenac, desipramine, econazole, ACE inhibitors, enalapril, ephedrine, epinephrine, epoetin and epoetin derivatives, morphinans, calcium antagonists, modafinil, orlistat, peptide antibiotics, phenytoin, riluzoles, risedronate, sildenafil, topiramate, estrogen, progestogen and progestogen derivatives, testosterone derivatives, androgen and androgen derivatives, ethenzamide, etofenamate, etofibrate, fenofibrate, etofylline, famciclovir, famotidine, felodipine, fentanyl, fenticonazole, gyrase inhibitors, fluconazole, fluarizine, fluoxetine, flurbiprofen, ibuprofen, fluvastatin, follitropin, formoterol, fosfomicin, furosemide, fusidic acid, gallopamil, ganciclovir, gemfibrozil, ginkgo, Saint John's wort, glibenclamide, urea derivatives as oral antidiabetics, glucagon, glucosamine and glucosamine derivatives, glutathione, glycerol and glycerol derivatives, hypothalamus hormones, guanethidine, halofantrine, haloperidol, heparin (and derivatives), hyaluronic acid, hydralazine, hydrochlorothiazide (and derivatives), salicylates, hydroxyzine, imipramine, indometacin, indoramine, insulin, iodine and iodine derivatives, isoconazole, isoprenaline, glucitol and glucitol derivatives, itraconazole, ketoprofen, ketotifen, lacidipine, lansoprazole, levodopa, levomethadone, thyroid hormones, lipoic acid (and derivatives), lisinopril, lisuride, lofepramine, loperamide, loratadine, maprotiline, mebendazole, mebeverine, meclozine, mefenamic acid, mefloquine, meloxicam, mepindolol, meprobamate, mesalazine, mesuximide, metamizole, metformin, methylphenidate, metixene, metoprolol, metronidazole, mianserin, miconazole, minoxidil, misoprostol, mizolastine, moexipril, morphine and morphine derivatives, evening primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline and adrenaline derivatives, novamine sulfone, noscapine, nystatin, olanzapine, olsalazine, omeprazole, omoconazole, oxaceprol, oxiconazole, oxymetazoline, pantoprazole, paracetamol (acetaminophen), paroxetine, penciclovir, pentazocine, pentifylline, pentoxifylline, perphenazine, pethidine, plant extracts, phenazone, pheniramine, barbituric acid derivatives, phenylbutazone, pimozide, pindolol, piperazine, piracetam, pirenzepine, piribedil, piroxicam, pramipexole, pravastatin, prazosin, procaine, promazine, propiverine, propranolol, propyphenazone, protionamide, proxyphylline, quetiapine, quinapril, quinaprilat, ramipril, ranitidine, reproterol, reserpine, ribavirin, risperidone, ritonavir, ropinirole, roxatidine, ruscogenin, rutoside (and derivatives), sabadilla, salbutamol, salmeterol, scopolamine, selegiline, sertaconazole, sertindole, sertralion, silicates, simvastatin, sitosterol, sotalol, spaglumic acid, spirapril, spironolactone, stavudine, streptomycin, sucralfate, sufentanil, sulfasalazine, sulpiride, sultiam, sumatriptan, suxamethonium chloride, tacrine, tacrolimus, taliolol, taurolidine, temazepam, tenoxicam, terazosin, terbinafine, terbutaline, terfenadine, terlipressin, tertatolol, teryzoline, theobromine, butizine, thiamazole, phenothiazines, tiagabine, tiapride, propionic acid derivatives, ticlopidine, timolol, tinidazole, tioconazole, tioguanine, tioxolone, tiropramide, tizanidine, tolazoline, tolbutamide, tolcapone, tolnaftate, tolperisone, topotecan, torasemide, tramadol, tramazoline, trandolapril, tranylcypromine, trapidil, trazodone, triamcinolone derivatives, triamterene, trifluperidol, trifluridine, trimipramine, tripelennamine, triprolidine, trifosfamide, tromantadine, trometamol, tropalpin, troxerutine, tulobuterol, tyramine, tyrothricin, urapidil, valaciclovir, valproic acid, vancomycin, vecuronium chloride, Viagra, venlafaxine, verapamil, vidarabine, vigabatrin, viloazine, vincamine, vinpocetine, viquidil, warfarin, xantinol nicotinate, xipamide, zafirlukast, zalcitabine, zidovudine, zolmitriptan, zolpidem, zoplicone, zotipine, amphotericin B, caspofungin, voriconazole, resveratrol, PARP-1 inhibitors (including imidazoquinolinone, imidazpyridine, and isoquinolindione, tissue plasminogen activator (tPA), melagatran, lanoteplase, reteplase, staphylokinase, streptokinase, tenecteplase, urokinase, abciximab (ReoPro), eptifibatide, tirofiban, prasugrel, clopidogrel, dipyridamole, cilostazol, VEGF, heparan sulfate, chondroitin sulfate, elongated "RGD" peptide binding domain, CD34 antibodies, cerivastatin, etorvastatin, losartan, valartan, erythropoietin, rosiglitazone, pioglitazone, mutant protein Apo A1 Milano, adiponectin, (NOS) gene therapy, glucagon-like peptide 1, atorvastatin, and atrial natriuretic peptide (ANP), lidocaine, tetracaine, dibucaine, hyssop, ginger, turmeric, Arnica montana, helenalin, cannabichromene, rofecoxib, hyaluronidase, and salts, derivatives, isomers, racemates, diastereoisomers, prodrugs, hydrate, ester, or analogs thereof.

In some embodiments, the coating has substantially uniform thickness and covers substantially the entire surface of said substrate.

In some embodiments, the coating comprises a microstructure. In some embodiments, pharmaceutical particles are sequestered or encapsulated within said microstructure. In some embodiments, the microstructure comprises microchannels, micropores and/or microcavities. In some embodiments, the microstructure is selected to allow sustained release of said at least one pharmaceutical agent. In some embodiments, the microstructure is selected to allow controlled release of said at least one pharmaceutical agent.

In some embodiments, the coating comprises at least two pharmaceutical agents. In some embodiments, the pharmaceutical agent is in the form of particles having an average diameter from 2 nm to 500 nm.

Provided herein is a method of depositing a coating onto a substrate, said coating comprising: at least one polymer comprising poly(D,L-lactic-co-glycolic acid) substantially free of acidic impurities; and at least one pharmaceutical agent in a therapeutically desirable morphology and/or at least one active biological agent; said method comprising the following steps: discharging the at least one pharmaceutical agent and/or at least one active biological agent in dry powder form through a first orifice; discharging the at least one polymer in dry powder form through a second orifice; depositing the polymer and pharmaceutical agent and/or active biological agent particles onto said substrate, wherein an electrical potential is maintained between the substrate and the polymer and pharmaceutical agent and/or active biological agent particles, thereby forming said coating; and sintering said coating under conditions that do not substantially modify the morphology of said pharmaceutical agent and/or the activity of said biological agent.

Provided herein is a method of depositing a coating onto a substrate, said coating comprising: at least one polymer comprising poly(D,L-lactic-co-glycolic acid) substantially free of acidic impurities; and at least one pharmaceutical agent in a therapeutically desirable morphology and/or at least one active biological agent;
said method comprising the following steps: discharging the at least one pharmaceutical agent and/or at least one active biological agent in dry powder form through a first orifice; forming a supercritical or near supercritical fluid solution comprising at least one supercritical fluid solvent and at least one polymer and discharging said supercritical or near supercritical fluid solution through a second orifice under conditions sufficient to form solid particles of the polymer; depositing the polymer and pharmaceutical agent and/or active biological agent particles onto said substrate, wherein an electrical potential is maintained between the substrate and the polymer and pharmaceutical agent and/or active biological agent particles, thereby forming said coating; and sintering said coating under conditions that do not substantially modify the morphology of said pharmaceutical agent and/or the activity of said biological agent.

Provided herein is a method of depositing a coating onto a substrate, said coating comprising: at least one polymer comprising poly(D,L-lactic-co-glycolic acid) substantially free of acidic impurities; and at least one pharmaceutical agent in a therapeutically desirable morphology in dry powder form and/or at least one active biological agent; said method comprising the following steps: discharging the at least one pharmaceutical agent and/or at least one active biological agent through a first orifice; forming a first stream of a polymer solution comprising at least one solvent and at least one polymer; forming a second stream of a supercritical or near supercritical fluid comprising at least one supercritical fluid; contacting said first and second streams, whereby said supercritical or near supercritical fluid acts as a diluent of said solution under conditions sufficient to form particles of said polymer; depositing the polymer and pharmaceutical agent and/or active biological agent particles onto said substrate, wherein an electrical potential is maintained between the substrate and the polymer and pharmaceutical agent and/or active biological agent particles, thereby forming said coating; and sintering said coating under conditions that do not substantially modify the morphology of said pharmaceutical agent and/or the activity of said biological agent.

In some embodiments, the at least one polymer comprising poly(D,L-lactic-co-glycolic acid) substantially free of acidic impurities is formed by any of the methods described herein. In some embodiments, the at least one polymer comprising poly(D,L-lactic-co-glycolic acid) substantially free of acidic impurities is one of the compositions described herein.

In some embodiments, the method further comprises depositing a top layer on said coating.

In some embodiments, the top layer is a polymer film.

The method of some embodiments is carried out in an open vessel. The method of some embodiments is carried out in a closed vessel.

In some embodiments, the first and said second orifices are provided as one single orifice.

In some embodiments, the polymer and said pharmaceutical agent and/or active biological agent are mixed together prior to discharging.

In some embodiments, the polymer and said pharmaceutical agent and/or active biological agent particles are discharged simultaneously.

In some embodiments, the polymer and said pharmaceutical agent and/or active biological agent are discharged in succession.

In some embodiments, the first and the second orifices are discharged to form a multilayer coating.

In some embodiments, the pharmaceutical agent and/or active biological agent is evenly dispersed throughout said coating.

In some embodiments, the pharmaceutical agent and/or active biological agent is not evenly dispersed throughout said coating.

The method of some embodiments further comprises discharging a third dry powder comprising a second pharmaceutical agent in a therapeutically desirable morphology in dry powder form and/or active biological agent whereby a coating comprising at least two different pharmaceutical agents and/or active biological agents is deposited on said substrate.

In some embodiments, the substrate is electrostatically charged.

In some embodiments, the substrate is a biomedical implant. In some embodiments, the biomedical implant is selected from the group consisting of stents, joints, screws, rods, pins, plates, staples, shunts, clamps, clips, sutures, suture anchors, electrodes, catheters, leads, grafts, dressings, pacemakers, pacemaker housings, cardioverters, cardioverter housings, defibrillators, defibrillator housings, prostheses, ear drainage tubes, ophthalmic implants, orthopedic devices, vertebral disks, bone substitutes, anastomotic devices, perivascular wraps, colostomy bag attachment devices, hemostatic barriers, vascular implants, vascular supports, tissue adhesives, tissue sealants, tissue scaffolds and intraluminal devices.

In some embodiments, the substrate is biodegradable. In some embodiments, the substrate and said coating are biodegradable.

In some embodiments, the therapeutically desirable morphology of said pharmaceutical agent is crystalline or semi-crystalline.

In some embodiments, at least 50% of said pharmaceutical agent in powder form is crystalline or semicrystalline.

In some embodiments, the pharmaceutical agent comprises at least one drug.

In some embodiments, the at least one drug is selected from the group consisting of antirestenotic agents, antidiabetics, analgesics, antiinflammatory agents, antirheumatics, antihypotensive agents, antihypertensive agents.

In some embodiments, the activity of said active biological agent is of therapeutic or prophylactic value.

In some embodiments, the biological agent is selected from the group comprising peptides, proteins, enzymes, nucleic acids, antisense nucleic acids, antimicrobials, vitamins, hormones, steroids, lipids, polysaccharides and carbohydrates.

In some embodiments, the activity of said active biological agent is influenced by the secondary, tertiary or quaternary structure of said active biological agent.

In some embodiments, the active biological agent possesses a secondary, tertiary or quaternary structure which is not substantially changed after the step of sintering said coating.

In some embodiments, the active biological agent further comprises a stabilizing agent.

In some embodiments, the sintering comprises treating said coated substrate with a compressed gas, compressed liquid or supercritical fluid that is a non-solvent for both the polymer and the pharmaceutical and/or biological agents.

In some embodiments, the compressed gas, compressed liquid or supercritical fluid comprises carbon dioxide, isobutylene or a mixture thereof.

In some embodiments, the at least one polymer comprises two or more polymers, wherein the first polymer swells in aqueous media and the second polymer does not substantially swell in aqueous media.

In some embodiments, in aqueous media the pharmaceutical agent and/or active biological agent elutes from said first polymer, and substantially does not elute from second polymer.

In some embodiments, the elution profile of said pharmaceutical agent and/or active biological agent is controllable by altering at least one parameter selected from the group consisting of the relative polymer amounts, the polymer particle sizes, the polymer particle shapes, the physical distribution of the polymers, the sintering conditions or any combination thereof.

Provided herein is a method for depositing a coating comprising a polymer and pharmaceutical agent on a substrate, wherein the polymer comprises poly(D,L-lactic-co-glycolic acid) substantially free of acidic impurities and wherein the method comprises: forming a supercritical or near critical fluid mixture that includes at least one polymer and at least one pharmaceutical agent discharging a spray of the supercritical or near critical fluid mixture through a constriction under conditions sufficient to form particles of the pharmaceutical agent and particles of the polymer that are substantially free of supercritical fluid solvent or solvents, wherein the constriction comprises an insulator material; providing a first electrode that is secured to the constriction and that can generate an electrical field for charging the solid pharmaceutical particles and/or the polymer particles to a first electric potential after they exit the constriction; depositing the charged solid pharmaceutical particles and polymer particles to form a coating onto said substrate; and sintering said coating under conditions that do not substantially modify the morphology of said solid pharmaceutical particles.

In some embodiments, the poly(D,L-lactic-co-glycolic acid) substantially free of acidic impurities is formed by any of the methods described herein.

In some embodiments, the first electrode is located adjacent the spray discharge from the constriction.

In some embodiments, the method comprises coupling a second electrode to the substrate that can charge the substrate to a second electric potential.

In some embodiments, the method comprises providing a chamber enclosing the discharged spray wherein the chamber comprises an insulator material.

In some embodiments, the coated substrates are produced at a rate of 10 or more substrates every hour.

A device comprising a substrate; a plurality of layers deposited on said stent framework to form said coronary stent; wherein at least one of said layers comprises a polymer comprising poly(D,L-lactic-co-glycolic acid) substantially free of acidic impurities and at least one of said layers comprises rapamycin; wherein at least part of rapamycin is in crystalline form and said rapamycin is provided at a reduced dose compared to a conventional drug eluting stent.

In some embodiments, the rapamycin and polymer are in the same layer; in separate layers or form overlapping layers.

In some embodiments, the plurality of layers comprise five layers deposited as follows: a first polymer layer, a first rapamycin layer, a second polymer layer, a second rapamycin layer and a third polymer layer.

In some embodiments, the substrate is a biomedical implant selected from the group consisting of stents, joints, screws, rods, pins, plates, staples, shunts, clamps, clips, sutures, suture anchors, electrodes, catheters, leads, grafts, dressings, pacemakers, pacemaker housings, cardioverters, cardioverter housings, defibrillators, defibrillator housings, prostheses, ear drainage tubes, ophthalmic implants, orthopedic devices, vertebral disks, bone substitutes, anastomotic devices, perivascular wraps, colostomy bag attachment devices, hemostatic barriers, vascular implants, vascular supports, tissue adhesives, tissue sealants, tissue scaffolds and intraluminal devices.

A device, comprising: a stent framework; and a rapamycin-polymer coating wherein at least part of rapamycin is in crystalline form and the rapamycin-polymer coating comprises one or more resorbable polymers and said rapamycin is provided at a reduced dose compared to a conventional drug eluting stent, and wherein the resorbable polymer comprises poly(D,L-lactic-co-glycolic acid) substantially free of acidic impurities.

In some embodiments, the rapamycin-polymer coating has substantially uniform thickness and rapamycin in the coating is substantially uniformly dispersed within the rapamycin-polymer coating.

In some embodiments, at least part of said rapamycin forms a phase separate from one or more phases formed by said polymer.

In some embodiments, the rapamycin is at least 50% crystalline. In some embodiments, the rapamycin is at least 75% crystalline. In some embodiments, the rapamycin is at least 90% crystalline. In some embodiments, the rapamycin is at least 95% crystalline. In some embodiments, the rapamycin is at least 99% crystalline.

In some embodiments, the polymer is a mixture of two or more polymers. In some embodiments, the mixture of polymers forms a continuous film around particles of rapamycin. In some embodiments, the two or more polymers are intimately mixed. In some embodiments, the mixture comprises no single polymer domain larger than about 20 nm. In some embodiments, each polymer in said mixture comprises a discrete phase.

In some embodiments, the discrete phases formed by said polymers in said mixture are larger than about 10nm. In some embodiments, the discrete phases formed by said polymers in said mixture are larger than about 50nm.

In some embodiments, the rapamycin in said stent has a shelf stability of at least 3 months.

In some embodiments, the rapamycin in said stent has a shelf stability of at least 6 months.

In some embodiments, the rapamycin in said stent has a shelf stability of at least 12 months.

In some embodiments, the coating is substantially conformal.

In some embodiments, the device provides an elution profile wherein about 10% to about 50% of rapamycin is eluted at week 1 after the composite is implanted in a subject under physiological conditions, about 25% to about 75% of rapamycin is eluted at week 2 and about 50% to about 100% of rapamycin is eluted at week 6.

In some embodiments, the device provides an elution profile wherein about 10% to about 50% of rapamycin is eluted at week 1 after the composite is implanted in a subject under physiological conditions, about 20% to about 75% of rapamycin is eluted at week 2 and about 50% to about 100% of rapamycin is eluted at week 10.

In some embodiments, the device provides an elution profile comparable to first order kinetics.

In some embodiments, the device provides elution profile control.

In some embodiments, the device provides tissue concentration control.

In some embodiments, the device provides tissue concentration of at least twice the tissue concentration provided by a conventional stent.

In some embodiments, the device provides tissue concentration of at least 5 times greater than the tissue concentration provided by a conventional stent.

In some embodiments, the device provides tissue concentration of at least 10 times greater than the tissue concentration provided by a conventional stent.

In some embodiments, the device provides tissue concentration of at least 15 times greater than the tissue concentration provided by a conventional stent.

In some embodiments, the device provides tissue concentration of at least 20 times greater than the tissue concentration provided by a conventional stent.

In some embodiments, the device provides tissue concentration of at least 50 times greater than the tissue concentration provided by a conventional stent.

In some embodiments, the device provides tissue concentration of at least 100 times greater than the tissue concentration provided by a conventional stent.

In some embodiments, the polymer is resorbed within 45-90 days after an angioplasty procedure.

In some embodiments, the device provides reduced inflammation over the course of polymer resorbtion compared to a conventional stent.

Provided herein is a method of preparing a coated device comprising: providing a substrate; depositing a plurality of layers on said substrate to form said coated device; wherein at least one of said layers comprises a drug-polymer coating wherein at least part of the drug is in crystalline form and the polymer is a bioabsorbable polymer comprising poly(D,L-lactic-co-glycolic acid) substantially free of acidic impurities.

In some embodiments, the substrate is a stent framework.

In some embodiments, the drug and polymer are in the same layer; in separate layers or in overlapping layers.

In some embodiments, the substrate is made of stainless steel. In some embodiments, the substrate is formed from a metal alloy. In some embodiments, the substrate is formed from a cobalt chromium alloy. In some embodiments, the substrate has a thickness of about 50% or less of a thickness of the coronary stent.

In some embodiments, the substrate has a thickness of about 100 µm or less.

In some embodiments, the method comprises depositing 4 or more layers. In some embodiments, the method comprises depositing 10, 20, 50, or 100 layers. In some embodiments, the method comprises depositing at least one of: at least 10, at least 20, at least 50, and at least 100 layers.

In some embodiments, the drug comprise a macrolide immunosuppressive (limus) drug. In some embodiments, the macrolide immunosuppressive drug comprises one or more of rapamycin, biolimus (biolimus A9), 40-0-(2-Hydroxyethyl)rapamycin (everolimus), 40-0-Benzyl-rapamycin, 40-0-(4'-Hydroxymethyl)benzyl-rapamycin, 40-O-[4'-(1,2-Dihydroxyethyl)]benzyl-rapamycin, 40-0-Allyl-rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin 40-O-(2-Hydroxy)ethoxycar-bonylmethyl-rapamycin, 40-O-(3-Hydroxy)propyl-rapamycin 4O-O-(6-Hydroxy)hexyl-rapamycin 40-O-[2-(2-Hydroxy)ethoxy]ethyl-rapamycin 4O-O-[(3S)-2,2-Dimethyldioxolan-3-yl]methyl-rapamycin, 40-O-[(2S)-2,3-Dihydroxyprop-1-yl]-rapamycin, 4O-O-(2-Acetoxy)ethyl-rapamycin 4O-O-(2-Nicotinoyloxy)ethyl-rapamycin, 4O-O-[2-(N-Morpholino)acetoxy]ethyl-rapamycin 4O-O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methyl-rapamycin, 4O-O-(2-Aminoethyl)-rapamycin, 4O-O-(2-Acetaminoethyl)-rapamycin 4O-O-(2-Nicotinamidoethyl)-rapamycin, 4O-O-(2-(N-Methyl-imidazo-2'-ylcarbethoxamido)ethyl)-rapamycin, 4O-O-(2-Ethoxycarbonylaminoethyl)-rapamycin, 40-0-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), and 42-[3-hydroxy-2-(hydroxymethyl)-2-methylpropanoate]rapamycin (temsirolimus), (42S)-42-Deoxy-42-(1H-tetrazol-1-yl)-rapamycin (zotarolimus), and salts, derivatives, isomers, racemates, diastereoisomers, prodrugs, hydrate, ester, or analogs thereof.

In some embodiments, the macrolide immunosuppressive drug is at least 50% crystalline.

In some embodiments, depositing a plurality of layers on said stent framework to form said coronary stent comprises depositing polymer particles on said framework by an RESS process. In some embodiments, depositing a plurality of layers on said stent framework to form said coronary stent comprises depositing polymer particles on said framework in dry powder form.

Provided herein is a coated stent, comprising: a stent framework; a first layer of bioabsorbable polymer; and a rapamycin-polymer coating comprising rapamycin and a second bioabsorbable polymer wherein at least part of rapamycin is in crystalline form and wherein the first polymer is a slow absorbing polymer and the second polymer is a fast absorbing polymer, and wherein at least one of the first polymer and the second polymer is poly(D,L-lactic-co-glycolic acid) substantially free of acidic impurities.

### INCORPORATION BY REFERENCE

All publications and patent applications mentioned in this specification are herein incorporated by reference to the same extent as if each individual publication or patent application was specifically and individually indicated to be incorporated by reference.

### DETAILED DESCRIPTION OF THE INVENTION

### Compositions

Hydrolytic breakdown of poly(alpha-hydroxycarboxylic acids) is accelerated by the presence of catalytic quantities of acid moieties. Poly(alpha-hydroxycarboxylic acids) are produced by polymerizing their dimeric six-membered cyclic ester condensate (1,4-dioxane-2,5-dione) or by polymerizing the alpha-hydroxycarboxylic acid (e.g. gycolic acid) itself into the multi-membered linear poly(alpha-hydroxycarboxylic acid). Either polymerization route leaves traces of the starting monomer, as the cyclic ester or the ring-opened carboxylic acid, and traces of oligomeric acidic fragments. Upon exposure to water the cyclic ester is readily hydrolysized to an hydroxycarboxylic acid (US Patents 3,457,280 and 3,597,449). Hence all manufactured poly(alpha-hydroxycarboxylic acids) contain small quantities of acid moieties that then contribute to its accelerated hydrolytic breakdown. The present invention discloses compositions without such acid moieties. In addition, it describes methods for producing such compositions by sequestering said catalytic quantities of acid moieties.

Provided herein is a composition comprising a poly(alpha-hydroxycarboxylic acid) substantially free of acidic impurities.

In one embodiment is a composition comprising poly(D,L-lactic-co-glycolic acid) substantially free of acidic impurities.

In another embodiment is a composition comprising poly(L-lactic acid) substantially free of acidic impurities.

In another embodiment is a composition comprising poly(D-lactic acid) substantially free of acidic impurities.

In another embodiment is a composition comprising poly(D,L-lactic acid) substantially free of acidic impurities.

The present invention provides compositions comprising biodegradable polymers with improved properties, specifically poly(D,L-lactic-co-glycolic acid) (PLGA). The acidic impurities present in the PLGA lower the pH of the micro-environment surrounding the PLGA when placed in contact with water. The pH reduction of the micro-environment accelerates the acid-catalysized hydrolysis of the ester bonds in the PLGA. Thus, small amounts of acidic impurties in the PLGA dramatically promote the degradation of the biopolymer. An additional aspect of the compositions of the invention is the enhanced degradation profile. The acid-free polymers described herein advantageously exhibit a reduced rate of degradation, but the induction period prior to the begining of hydrolysis is also extended.

In one embodiment of the invention is a composition comprising poly(D,L-lactic-co-glycolic acid) substantially free of acid impurities. In another embodiment, the composition contains less than 0.1% (w/w) of acidic impurity. In another embodiment, the composition contains less than 0.2% (w/w) of acidic impurity. In another embodiment, the composition contains less than 0.3% (w/w) of acidic impurity. In another embodiment, the composition contains less than 0.4% (w/w) of acidic impurity. In another embodiment, the composition contains less than 0.5% (w/w) of acidic impurity. In another embodiment, the composition contains less than 0.6% (w/w) of acidic impurity. In another embodiment, the composition contains less than 0.7% (w/w) of acidic impurity. In another embodiment, the composition contains less than 0.8% (w/w) of acidic impurity. In another embodiment, the composition contains less than 0.9% (w/w) of acidic impurity. In another embodiment, the composition contains less than 1.0 % (w/w) of acidic impurity. In another embodiment, the composition contains less than 1.1% (w/w) of acidic impurity. In another embodiment, the composition contains less than 1.2% (w/w) of acidic impurity. In another embodiment, the composition contains less than 1.3% (w/w) of acidic impurity. In another embodiment, the composition contains less than 1.4% (w/w) of acidic impurity. In another embodiment, the composition contains less than 1.5% (w/w) of acidic impurity.

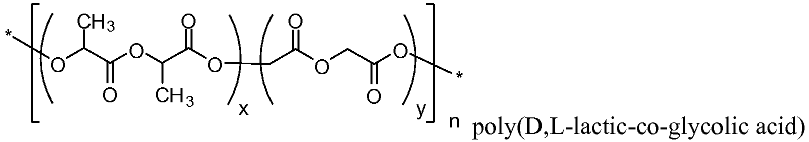

In another embodiment, the poly(D,L-lactic-co-glycolic acid) has a ratio of lactic acid monomer to glycolic acid monomer ranging from 82:18 to 88:12. In another embodiment, the poly(D,L-lactic-co-glycolic acid) has a ratio of lactic acid monomer to glycolic acid monomer ranging from 72:28 to 78:22. In another embodiment, the poly(D,L-lactic-co-glycolic acid) has a ratio of lactic acid monomer to glycolic acid monomer ranging from 62:38 to 68:32. In another embodiment, the poly(D,L-lactic-co-glycolic acid) has a ratio of lactic acid monomer to glycolic acid monomer ranging from 47:53 to 53:47. In another embodiment, the poly(D,L-lactic-co-glycolic acid) has a weight average molecular weight of about 5,000, of about 6,000, of about 7,000, of about 8,000, of about 9,000, of about 10,000, of about 11,000, of about 12,000, of about 13,000, of about 14,000, of about 15,000, of about 16,000, of about 17,000, of about 18,000, of about 19,000, of about 20,000, of about 21,000, of about 22,000, of about 23,000, of about 24,000, of about 25,000, of about 30,000, of about 35,000, of about 40,000, of about 45,000, of about 50,000, of about 55,000, of about 60,000, of about 65,000, of about 70,000, of about 75,000, of about 80,000, of about 85,000, of about 90,000, of about 95,000, or of about 100,000. In one embodiment, the carboxy terminus of the poly(D,L-lactic-co-glycolic acid) is capped with an alkyl ester. In a further embodiment, the carboxy terminus of the poly(D,L-lactic-co-glycolic acid) is capped with a methyl ester. In an additional embodiment, the carboxy terminus of the poly(D,L-lactic-co-glycolic acid) is capped with an ethyl ester.

The compositions comprising poly(D,L-lactic-co-glycolic acid) substantially free of acid impurities display enhanced properties when compared to compositions comprising poly(D,L-lactic-co-glycolic acid) containing acid impurities. In particular, the compositions free of acid impurities show a reduced rate of hydrolysis of the biodegradable polymer and thus a longer useful time period for controlled-release applications and structural applications. Additionally, the compositions free of acid impurities show a reduced tendency to induce an inflammatory response and are therefore more biocompatibile. Additional desirable properties of the compositions free of acid impurities include an enhanced shelf-life that may be due for example to a reduced rate of background hydrolysis upon inadverent exposure to moisture due for example to packaging. Another advantage relates to providing fast-absorbing compositions with a more stable induction time to loss of mechanical properties.

### Methods of sequestering catalytic quantities of acid moieties in poly(alpha-hydroxycarboxylic acids)

In one embodiment is a method for the preparation of compositions comprising poly(alpha-hydroxycarboxylic acids) substantially free of acid impurities. In another embodiment, the acid moieties within the poly(alpha-hydroxycarboxylic acid) diffuse and contact a dry, insoluble, solid base that reacts with said acid moiety and creates a salt that is insoluble in the remainder of the polymer or solutions thereof. The polymer may be in any one of a number of phases: [a] solvent-free solution phase, at or above ambient temperature; [b] solution phase, at normal atmospheric pressure, in an organic solvent that does not chemically react with the solid base; [c] solution phase in a suitable solvent for the creation of a supercritical fluid state, or [d] solid phase.

The solid base employed in the methods described herein are generally considered safe for use in therapeutic applications. Examples include bases derived from metal cations of elements such as Li, Na, K, Mg, Ca, B and Al. In one embodiment, the solid base is selected from the group consisting of: MgO, CaO, LiH, LiAlH₄, NaH, NaBH₄, KH, MgH₂, and CaH₂. In another embodiment, the solid base is CaH₂.

One method for the removal of acidic impurties, when the molecular weight and viscosity is low enough, is to pass the acid catalyst-containing poly(alpha-hydroxycarboxylic acid) through a column of a solid base, such as CaH₂, that is not soluble in the polymer. In some embodiments, the column of CaH₂ is heated to maintain the poly(-hydroxycarboxylic acid) in a liquid state. The small acid molecules upon contacting the CaH₂ will immediately react and form an insoluble calcium salt. Hence, the small acid molecules will be sequestered and removed from the bulk polymer. The acid free poly(alpha-hydroxycarboxylic acid) material can then be further manipulated into the desired product. In one such application, the acid free material can be injection molded into a bone pin or other biodegradable-controlled device.

Another method for the removal of acidic impurties, when solubility in an organic solvent allows, is to form a solution in a solvent such as tetrahydrofuran, dichloromethane, dimethyl sulfoxide, toluene or dimethy formamide. The polymer solution is then treated under anhydrous conditions with a small quantity of solid base, such as LiAlH₄ or CaH₂ Any water or acid catalyst present in this suspension will react with the hydride and form H₂ gas. The acid will be converted to the Li or Ca salt. The suspension is then filtered to remove excess hydride. The resultant acid-free solution can then be used. One such use is to coat a biodegradable-controlled prosthetic device. Sodium borohydride on basic alumina can also be used in place of the LiAlH₄. This supported reagent is easier to handle and filter than the free hydride.

Another method involves the use of supercritical fluid technology. A 50:50 copolymer of lactic and glycolic acid, with a Mn average molecular weight of about 20,000 daltons, is readily dissolved in a chamber charged with 1,1,1,2,3,3- hexafluoropropane (FC236) at sufficient pressure to induce the supercritical state. Liquid and compressed liquid conditions are alsoo contemplated whereby the copolymer is soluble. This chamber is fitted with a magnetic stirrer that allows the polymer to be quickly dissolved and, once dissolved, to be circulated against the outer wall of the chamber which is made of a fine 304 stainless steel woven wire cloth/screen (635 x 635 mesh; 0.0009" wire diameter; with a percentage of open area range of 15% ± 5%) that has entrapped behind it a slight excess of CaH₂. The CaH₂ is in the form of course granules (2-5 mm) that cannot pass through the screen. The solution is readily permeable and, therefore, the catalytic quantities of acid moieties come in contact with the CaH₂ behind the screen but not in the main chamber itself. When such contact is made, the acid is converted to the Ca salt that precipitates onto the CaH₂ or the woven wire mess and thus, the original catalytic quantities of acid moieties are sequestered and removed from the original solution. During this process, the H₂ gas generated becomes a component of the supercritical fluid. The solution within the CaH₂ zone now has a reduced concentration of acid moieties and is free to reenter the main chamber only to be replaced by more solution with a higher concentration of acid moieties. Within 6 hours, the concentration of catalytic quantities of acid moieties is no longer detectable. In some embodiments, the supercritical fluid solution of poly(D,L-lactic-co-glycolic acid) is exposed to solid base for a period of 2 to 6 hours. In other embodiments, supercritical fluid solution of poly(D,L-lactic-co-glycolic acid) is exposed to solid base for a period of 6 to 10 hours. In other embodiments, supercritical fluid solution of poly(D,L-lactic-co-glycolic acid) is exposed to solid base for a period of 10 to 24 hours. The acid free, supercritical fluid solution of poly(D,L-lactic-co-glycolic acid) can now be used in the production of a biomedical device. In one embodiment, the supercritical fluid solution of poly(D,L-lactic-co-glycolic acid) can be dispensed directly from the supercritical fluid chamber. In one such applicaiton, the solution is heated to the appropriate temperature and sprayed on to a biodegradable-controlled prosthetic device such as an intracoronary stent.

A method that depends on purification in the solid state begins with fabricating poly(alpha-hydroxycarboxylic acids) into thin solid membranes by applying adequate pressure and temperature. Likewise most powders such as CaO, CaH₂ or MgO (with or with out binders) can also be pressed into flat sheets using a Carver Press (or the like) with smooth stainless steel platens. The two sets of sheets can then be interdigitized and pressed together to expel any entrapped air between them. This method is particularly effective for long storage times. Diffusion or migration of the catalytic quantities of acid moieties toward the solid base is relatively slow. But the activity at the interface is very close to zero and, hence, is an effective driving force. The dry stored sheets or films can then be remolded or dissolved in appropriate solvent for conversion into a useful device. The sequestering process can be accelerated by storing the interdigitized sheets in a dry incubator at 37 °C.

An additional method involves the use of electrophoresis. Electrophoretic transport of a low molecular weight, highly polar, acid molecule through a membrane of the proper pore size is much more facile than a high molecular weight, much less polar, neutral polymer. In spite of the many physical arrangments for the apparatus, and regardless of the medium through which molecules are allowed to migrate, all electrophoretic separations depend upon the charge distribution of the molecules being separated. The charge differential between a low molecular weight, highly polar, acid molecule and a high molecular weight, much less polar, neutral polymer is large and serves as the driving force for the electrophoresis method.

One dimensional electrophoresis is used for most routine protein and nucleic acid separations. The support medium for electrophoresis is a flat sheet of a polyacrylamide gel. This gel is formed from the polymerization of acrylamide and N,N-methylene-bisacrylamide. The separation of molecules within a gel is driven by the relative size of the pores formed within the gel. The pore size of a gel is determined by two factors, the total amount of acrylamide present (designated as %T) and the amount of cross-linker (%C). As the total amount of acrylamide increases, the pore size decreases. Gels of >15%T and > 7.5%C are used to limit the amount of poly(alpha-hydroxycarboxylic acids) that can be driven into the gel, and maximize the amount of catalytic quantities of acid moieties that can penetrate the gel.

In an additional embodiment are methods for the preparation of compositions comprising poly(D,L-lactic-co-glycolic acid) substantially free of acid impurities. In one embodiment, the method for the preparation of compositions comprising poly(D,L-lactic-co-glycolic acid) substantially free of acid impurities comprises contacting poly(D,L-lactic-co-glycolic acid) containing acidic impurties with a solid base; forming a salt of the acidic impurity; and separating the poly(D,L-lactic-co-glycolic acid) from the salt of the acidic impurity. In one embodiment, the solid base is selected from the group consisting of: MgO, LiH, NaH, KH, MgH₂, and CaH₂. In another embodiment, the solid base is CaH₂.

In another embodiment is a method for the preparation of a composition comprising poly(D,L-lactic-co-glycolic acid) substantially free of acidic impurities, said method comprising: dissolving the poly(D,L-lactic-co-glycolic acid) containing acidic impurties in an inert solvent; contacting poly(D,L-lactic-co-glycolic acid) solution with a metal hydride; forming a metal salt of the acidic impurity; and separating the poly(D,L-lactic-co-glycolic acid) from the metal salt of the acidic impurity. In one embodiment, the solid base is selected from the group consisting of: MgO, LiH, NaH, KH, MgH₂, and CaH₂. In another embodiment, the solid base is CaH₂. In one embodiment, the metal salt of the acidic impurity is separated from the poly(D,L-lactic-co-glycolic acid) by filtration. In another embodiment, the metal salt of the acidic impurity is separated from the poly(D,L-lactic-co-glycolic acid) by diffusion through a semi-permeable membrane. In another embodiment the method is performed in a supercritical state. In another embodiment the inert solvent is a fluorocarbon. In another embodiment, the fluorocarbon solvent is FC236.

### Coated Devices and Coating Methods

Provided herein are substrates coated with polymer compositions described herein and a pharmaceutical or biological agent in powder form. Provided herein are methods for depositing polymer compositions described herein and a pharmaceutical or biological agent in powder form onto a substrate.

Applicants specifically intend that all United States patent references cited herein be incorporated herein by reference in their entirety.

The present invention provides a cost-effective, efficient method for depositing a combination of an inert polymer or polymers and a pharmaceutical or biological agent or agents, onto parts or all surfaces of a substrate, to form a coating that is of a pre-determined, desired thickness, conformal, substantially defect-free, and uniform and the composition of the coating can be regulated. In particular, the present invention addresses the problem of existing coating processes, which do not allow for structural and morphological preservation of the agents deposited during the coating process.

One aspect of the invention entails the deposition of the pharmaceutical or biological agents as dry powders, using electrostatic capture to attract the powder particles to the substrate. Dry powder spraying is well known in the art, and dry powder spraying coupled with electrostatic capture has been described, for example in US Patents 5,470,603; 6,319,541; or 6,372,246. The deposition of the polymer can be performed in any number of standard procedures, as the morphology of the polymer, so long as it provides coatings possessing the desired properties (e.g. thickness, conformity, defect-free, uniformity etc), is of less importance. The function of the polymer is primarily one of inert carrier matrix for the active components of the coating.

In one aspect, the coating process involves taking the substrates that have been coated with pharmaceutical or biological agents and polymers and subjecting them to a sintering process that takes place under benign conditions, which do not significantly affect the structural and morphological integrity of the pharmaceutical and biological agents. The sintering process as used in the current invention refers to the process by which parts of the matrix or the entire polymer matrix becomes continuous (e.g., formation of a continuous polymer film). As discussed below, the sintering process is controlled to produce a fully conformal continuous matrix (complete sintering) or to produce regions or domains of continuous coating while producing voids (discontinuities) in the matrix. As well, the sintering process is controlled such that some phase separation is obtained between polymer different polymers (e.g., polymers A and B) and/or to produce phase separation between discrete polymer particles. The sintering process also improves the adhesion of the polymer coating. The sintering process involves treatment of the coated substrate with a compressed gas, compressed liquid, or supercritical fluid at conditions (e.g. temperature and pressure) such that it is a poor solvent or in some instances a non-solvent for the polymers, the pharmaceutical agents and the biological agents, but induces the formation of a continuous coating of polymer. The sintering process takes place under conditions (e.g. mild temperatures), and using benign fluids (e.g. a compressed gas, or supercritical fluid, the gas or fluid may comprise carbon dioxide, isobutylene or a mixture thereof for example) which will not significantly affect the structural and morphological integrity of the pharmaceutical and/or biological agents. It is noted that while under some situations better sintering results may be obtained by using supercritical or near critical fluids, in many embodiments according to the invention, treatment with compressed gas will provide the desired sintered polymer coating. Those of skill in the art will have no difficulty selecting a supercritical fluid, a near critical fluid or compressed gas in practicing the present invention. Sintering conditions may be adjusted such that the sintering process is not fully completed. That is, the sintering does not result in the formation of a fully continuous polymer matrix. When incomplete sintering is practiced according to the invention, some domains in the polymer matrix may be continuous, while other domains will define voids, cavities, pores, channels or interstices where the drug can be encapsulated or sequestered within the polymer matrix. Such a polymer matrix would be at a density less than the bulk density of the polymer; caused by micro or macroscopic voids in the polymer matrix. Alternatively, such a polymer matrix could retain phase separation of the polymer domains or in the case where multiple polymers are used, phase separation between the different polymer species. In most embodiments, whether the sintering process is complete or incomplete, the sintering conditions are selected to produce good adhesion of the coating to the substrate. For stents, adequate adhesion properties will generally reduce or prevent flaking or detachment of the coating from the stent during manipulation in use.

One aspect of the invention is the combination of two or more of the dry powder, RESS and SEDS spraying techniques.

Another aspect of the invention involves the dry powder spraying of a pharmaceutical agent, in a preferred particle size and morphology, into the same capture vessel as a polymer that is also dry powder sprayed, whereby the spraying of the agent and the polymer is sequential or simultaneous.

Another specific aspect of the invention involves the dry powder spraying of an active biological agent, in a preferred particle size and possessing a particular activity, into the same capture vessel as a polymer that is also dry powder sprayed, whereby the spraying of the agent and the polymer is sequential or simultaneous.

Yet another aspect of the invention involves the dry powder spraying of a pharmaceutical agent, in a preferred particle size and morphology, into the same capture vessel as a polymer that is sequentially or simultaneously sprayed by the RESS spray process.

Yet another aspect of the invention involves the dry powder spraying of an active biological agent, in a preferred particle size and possessing a particular activity, into the same capture vessel as a polymer that is sequentially or simultaneously sprayed by the RESS spray process.

Yet another aspect of the invention involves the dry powder spraying of a pharmaceutical agent, in a preferred particle size and morphology, into the same capture vessel as a polymer that is sequentially or simultaneously sprayed by the SEDS spray process.

Yet another aspect of the invention involves the dry powder spraying of an active biological agent, in a preferred particle size and possessing a particular activity, into the same capture vessel as a polymer that is sequentially or simultaneously sprayed by the SEDS spray process.

Any combination of the above six processes is contemplated by this aspect of the invention.

In further aspects of the invention the substrates that have been coated with pharmaceutical or biological agents and polymers, as described in the above embodiments are then subjected to a sintering process. The sintering process takes place under benign conditions, which do not affect the structural and morphological integrity of the pharmaceutical and biological agents, and refers to a process by which the co-deposited pharmaceutical agent or biological agent-polymer matrix, becomes continuous and adherent to the substrate. This is achieved by treating the coated substrate with a compressed gas, compressed liquid or supercritical fluid at conditions such that it is a poor solvent of the polymers, a weak solvent of the polymers or a non-solvent for the polymers, the pharmaceutical agents and the biological agents, but an agent suitable for the treatment of polymer particles to form continuous polymer coatings. The sintering process takes place under conditions (e.g. mild temperatures), and using benign fluids (e.g. supercritical carbon dioxide) which will not affect the structural and morphological integrity of the pharmaceutical and biological agents. Other sintering processes, which do not affect the structural and morphological integrity of the pharmaceutical and biological agents may also be contemplated by the present invention.

In further aspects of the invention, it is desirable to create coatings such that release of an active substance occurs with a predetermined elution profile when placed in the desired elution media. Coating properties can be modified in a variety of different ways in order to provide desirable elution profiles.

The chemical composition of the polymers can be varied, to provide greater or lesser amounts of polymers that will allow or restrict the elution of active substance. For example, if the intended elution media contain water, a higher content of polymers that swell in water, will allow for a faster elution of active substance. Conversely, a higher content of polymers that do not swell in aqueous media will result in a slower elution rate.

The coating properties can also be controlled by alternating polymer layers. Layers of polymers of different properties are deposited on the substrate in a sequential manner. By modifying the nature of the polymer deposited in each layer (e.g., depositing layers of different polymers) the elution profile of the coating is altered. The number of layers and the sequence in their deposition provide additional avenues for the design of coatings having controlled elution profiles.

The coating properties can also be modified by control of the macro and/or micro-structure of the polymer coating (diffusion pathways). This may be achieved by varying the coating process(es) or by using different sintering conditions.

The present invention provides several approaches for controlling the elution of a drug or several drugs. For example, in one embodiment, controlled elution is achieved by the segregation of different polymers (e.g. PEVA / PBMA). In another embodiment, control of elution is achieved by controlling the conditions during the sintering process such that controlled incomplete sintering of the polymer matrix is obtained, whereby the coating would retain some of the particle-like structure of the polymer particles as deposited. Incomplete sintering would provide pores/voids in the coating and allow additional pathways for elution of the drug, including drug elution around the polymer(s) instead of, or in addition to, elution through the polymer(s). The size of the pores or voids obtained through incomplete sintering of the polymer matrix may be obtained through several methods. For example, the rate of depressurization of a vessel in which the sintering process is carried out provides one avenue for controlling pore size. The size of the cavities or pores in the coating can be controlled by employing a porogen as an excipient and subsequent removal of at least a portion of the porogen, for example by treatment with a solvent of the porogen. Preferably, the porogen solvent comprises a densified gas (e.g.; carbon). In some embodiments the porogen is an SOA or other such hydrophobically derivatized carbohydrate. Removal of at least a portion of the porogen is preferably carried out during the sintering process.

In some aspects of the invention, the active substance elution profile is controllable by altering the polymer particle size. The method by which the polymer particles are deposited onto the substrate is thus varied to provide the desired elution rate. For example, for polymers released simultaneously through the same nozzle, RESS release from a supercritical solution would typically result in small polymer particles; RESS-like release from a mixture in a compressed gas usually generates larger polymer particles. Using the SEDS process can result in variable polymer particle size, depending on the particular SEDS conditions employed.

In further aspects of the invention, the active substance elution profile is controllable by altering the polymer particle shape. One way to achieve variation in polymer particle shape is to alter the initial concentration of the polymers. At lower initial concentrations, polymers are deposited as small particles. At increased concentrations, larger particles are formed. At higher concentrations, the formed particles become elongated, until at high concentrations the elongated features become fiber-like and eventually become continuous fibers.

In yet other aspects of the invention, the active substance elution profile is controllable by creating discrete domains of chemically different polymers. As described above, chemically different polymers will allow or restrict the elution of active substance in different elution media. By changing the position of such polymers in discrete macroscopic domains within the coating, the elution profiles will be adjustable. For example during a process whereby two different polymers are released sequentially through the same nozzle, particles of either polymer could be deposited to position them, for example, closer to the outside, the inside or the middle of the coating on the substrate. In another embodiment, the two polymers may be released simultaneously through two different nozzles at differing and/or alternating deposition rates, resulting in a similar effect. In a further embodiment, the deposition of eluting and non-eluting polymers is alternated to result in a fluctuating type of release. In yet other embodiments, the polymers are deposited to provide for a pulsatile release of active substance. Separation of the polymer(s) providing different domains for drug diffusion is achieved, for example, by subsequent spray of the polymers through same nozzle or by using multiple nozzles. Also, as described above, controlling the elution of the active substance may be achieved by layering of different polymers across the depth of the coating. A combination of domain separation and cross-depth layering is also contemplated for the design of coatings having controlled elution properties.

The deposition of active substance during any of these processes may be constant to provide even distribution throughout the coating, or the spraying of the active substance may be varied to result in differing amounts of active substance in the differing polymeric domains within the coating.

In further aspects of the invention, the active substance elution profile is controllable by varying the coating sintering conditions. For example, incomplete sintering will create open spaces, or pores in the interstitial spaces between the polymer particles, which will enable faster eluting of active substance from the coating. Another way to utilize the sintering conditions for elution control would be to deliberately create irregular coatings by foaming during the sintering process. Rapid pressure release of a CO₂- or isobutylene-impregnated polymer film induces formation of foamed polymers which would create a coating with increased porosity and be very 'open' to diffusion/elution. Thus the elution profile would be controllable by manipulating the foaming conditions, which in turn controls the pore density and size.

Applicants specifically intend that all United States patent references cited herein be incorporated herein by reference in their entirety.

The present invention provides a cost-effective, efficient method for depositing a combination of an inert polymer or polymers and a pharmaceutical or biological agent or agents, onto parts or all surfaces of a substrate, to form a coating that is of a pre-determined, desired thickness, conformal, substantially defect-free, and uniform and the composition of the coating can be regulated. In particular, the present invention addresses the problem of existing coating processes, which do not allow for structural and morphological preservation of the agents deposited during the coating process.

One aspect of the invention entails the deposition of the pharmaceutical or biological agents as dry powders, using electrostatic capture to attract the powder particles to the substrate. Dry powder spraying is well known in the art, and dry powder spraying coupled with electrostatic capture has been described, for example in US Patents 5,470,603; 6,319,541; or 6,372,246. The deposition of the polymer can be performed in any number of standard procedures, as the morphology of the polymer, so long as it provides coatings possessing the desired properties (e.g. thickness, conformity, defect-free, uniformity etc), is of less importance. The function of the polymer is primarily one of inert carrier matrix for the active components of the coating.

In one aspect, the coating process involves taking the substrates that have been coated with pharmaceutical or biological agents and polymers and subjecting them to a sintering process that takes place under benign conditions, which do not significantly affect the structural and morphological integrity of the pharmaceutical and biological agents. The sintering process as used in the current invention refers to the process by which parts of the matrix or the entire polymer matrix becomes continuous (e.g., formation of a continuous polymer film). As discussed below, the sintering process is controlled to produce a fully conformal continuous matrix (complete sintering) or to produce regions or domains of continuous coating while producing voids (discontinuities) in the matrix. As well, the sintering process is controlled such that some phase separation is obtained between polymer different polymers (e.g., polymers A and B) and/or to produce phase separation between discrete polymer particles. The sintering process also improves the adhesion of the polymer coating. The sintering process involves treatment of the coated substrate with a compressed gas, compressed liquid, or supercritical fluid at conditions (e.g. temperature and pressure) such that it is a poor solvent or in some instances a non-solvent for the polymers, the pharmaceutical agents and the biological agents, but induces the formation of a continuous coating of polymer. The sintering process takes place under conditions (e.g. mild temperatures), and using benign fluids (e.g. a compressed gas, or supercritical fluid, the gas or fluid may comprise carbon dioxide, isobutylene or a mixture thereof for example) which will not significantly affect the structural and morphological integrity of the pharmaceutical and/or biological agents. It is noted that while under some situations better sintering results may be obtained by using supercritical or near critical fluids, in many embodiments according to the invention, treatment with compressed gas will provide the desired sintered polymer coating. Those of skill in the art will have no difficulty selecting a supercritical fluid, a near critical fluid or compressed gas in practicing the present invention. Sintering conditions may be adjusted such that the sintering process is not fully completed. That is, the sintering does not result in the formation of a fully continuous polymer matrix. When incomplete sintering is practiced according to the invention, some domains in the polymer matrix may be continuous, while other domains will define voids, cavities, pores, channels or interstices where the drug can be encapsulated or sequestered within the polymer matrix. Such a polymer matrix would be at a density less than the bulk density of the polymer; caused by micro or macroscopic voids in the polymer matrix. Alternatively, such a polymer matrix could retain phase separation of the polymer domains or in the case where multiple polymers are used, phase separation between the different polymer species. In most embodiments, whether the sintering process is complete or incomplete, the sintering conditions are selected to produce good adhesion of the coating to the substrate. For stents, adequate adhesion properties will generally reduce or prevent flaking or detachment of the coating from the stent during manipulation in use.

One aspect of the invention is the combination of two or more of the dry powder, RESS and SEDS spraying techniques.

Another aspect of the invention involves the dry powder spraying of a pharmaceutical agent, in a preferred particle size and morphology, into the same capture vessel as a polymer that is also dry powder sprayed, whereby the spraying of the agent and the polymer is sequential or simultaneous.

Another specific aspect of the invention involves the dry powder spraying of an active biological agent, in a preferred particle size and possessing a particular activity, into the same capture vessel as a polymer that is also dry powder sprayed, whereby the spraying of the agent and the polymer is sequential or simultaneous.

Yet another aspect of the invention involves the dry powder spraying of a pharmaceutical agent, in a preferred particle size and morphology, into the same capture vessel as a polymer that is sequentially or simultaneously sprayed by the RESS spray process.

Yet another aspect of the invention involves the dry powder spraying of an active biological agent, in a preferred particle size and possessing a particular activity, into the same capture vessel as a polymer that is sequentially or simultaneously sprayed by the RESS spray process.

Yet another aspect of the invention involves the dry powder spraying of a pharmaceutical agent, in a preferred particle size and morphology, into the same capture vessel as a polymer that is sequentially or simultaneously sprayed by the SEDS spray process.

Yet another aspect of the invention involves the dry powder spraying of an active biological agent, in a preferred particle size and possessing a particular activity, into the same capture vessel as a polymer that is sequentially or simultaneously sprayed by the SEDS spray process.

Any combination of the above six processes is contemplated by this aspect of the invention.

In further aspects of the invention the substrates that have been coated with pharmaceutical or biological agents and polymers, as described in the above embodiments are then subjected to a sintering process. The sintering process takes place under benign conditions, which do not affect the structural and morphological integrity of the pharmaceutical and biological agents, and refers to a process by which the co-deposited pharmaceutical agent or biological agent-polymer matrix, becomes continuous and adherent to the substrate. This is achieved by treating the coated substrate with a compressed gas, compressed liquid or supercritical fluid at conditions such that it is a poor solvent of the polymers, a weak solvent of the polymers or a non-solvent for the polymers, the pharmaceutical agents and the biological agents, but an agent suitable for the treatment of polymer particles to form continuous polymer coatings. The sintering process takes place under conditions (e.g. mild temperatures), and using benign fluids (e.g. supercritical carbon dioxide) which will not affect the structural and morphological integrity of the pharmaceutical and biological agents. Other sintering processes, which do not affect the structural and morphological integrity of the pharmaceutical and biological agents may also be contemplated by the present invention.

In further aspects of the invention, it is desirable to create coatings such that release of an active substance occurs with a predetermined elution profile when placed in the desired elution media. Coating properties can be modified in a variety of different ways in order to provide desirable elution profiles.

The chemical composition of the polymers can be varied, to provide greater or lesser amounts of polymers that will allow or restrict the elution of active substance. For example, if the intended elution media contain water, a higher content of polymers that swell in water, will allow for a faster elution of active substance. Conversely, a higher content of polymers that do not swell in aqueous media will result in a slower elution rate.

The coating properties can also be controlled by alternating polymer layers. Layers of polymers of different properties are deposited on the substrate in a sequential manner. By modifying the nature of the polymer deposited in each layer (e.g., depositing layers of different polymers) the elution profile of the coating is altered. The number of layers and the sequence in their deposition provide additional avenues for the design of coatings having controlled elution profiles.

The coating properties can also be modified by control of the macro and/or micro-structure of the polymer coating (diffusion pathways). This may be achieved by varying the coating process(es) or by using different sintering conditions.

The present invention provides several approaches for controlling the elution of a drug or several drugs. For example, in one embodiment, controlled elution is achieved by the segregation of different polymers (e.g. PEVA / PBMA). In another embodiment, control of elution is achieved by controlling the conditions during the sintering process such that controlled incomplete sintering of the polymer matrix is obtained, whereby the coating would retain some of the particle-like structure of the polymer particles as deposited Incomplete sintering would provide pores/voids in the coating and allow a additional pathways for elution of the drug , including drug elution around the polymer(s) instead of or in addition to elution through the polymer(s). The size of the pores or voids obtained through incomplete sintering of the polymer matrix may be obtained through several methods. For example, the rate of depressurization of a vessel in which the sintering process is carried out provides one avenue for controlling pore size. The size of the cavities or pores in the coating can be controlled by employing a porogen as an excipient and subsequent removal of at least a portion of the porogen, for example by treatment with a solvent of the porogen. Preferably, the porogen solvent comprises a densified gas (e.g.; carbon). In some embodiments the porogen is an SOA or other such hydrophobically derivatized carbohydrate. Removal of at least a portion of the porogen is preferably carried out during the sintering process.

In some aspects of the invention, the active substance elution profile is controllable by altering the polymer particle size. The method by which the polymer particles are deposited onto the substrate is thus varied to provide the desired elution rate. For example, for polymers released simultaneously through the same nozzle, RESS release from a supercritical solution would typically result in small polymer particles; RESS-like release from a mixture in a compressed gas usually generates larger polymer particles. Using the SEDS process can result in variable polymer particle size, depending on the particular SEDS conditions employed.

In further aspects of the invention, the active substance elution profile is controllable by altering the polymer particle shape. One way to achieve variation in polymer particle shape is to alter the initial concentration of the polymers. At lower initial concentrations, polymers are deposited as small particles. At increased concentrations, larger particles are formed. At higher concentrations, the formed particles become elongated, until at high concentrations the elongated features become fiber-like and eventually become continuous fibers.

In yet other aspects of the invention, the active substance elution profile is controllable by creating discrete domains of chemically different polymers. As described above, chemically different polymers will allow or restrict the elution of active substance in different elution media. By changing the position of such polymers in discrete macroscopic domains within the coating, the elution profiles will be adjustable. For example during a process whereby two different polymers are released sequentially through the same nozzle, particles of either polymer could be deposited to position them, for example, closer to the outside, the inside or the middle of the coating on the substrate. In another embodiment, the two polymers may be released simultaneously through two different nozzles at differing and/or alternating deposition rates, resulting in a similar effect. In a further embodiment, the deposition of eluting and non-eluting polymers is alternated to result in a fluctuating type of release. In yet other embodiments, the polymers are deposited to provide for a pulsatile release of active substance. Separation of the polymer(s) providing different domains for drug diffusion is achieved, for example, by subsequent spray of the polymers through same nozzle or by using multiple nozzles. Also, as described above, controlling the elution of the active substance may be achieved by layering of different polymers across the depth of the coating. A combination of domain separation and cross-depth layering is also contemplated for the design of coatings having controlled elution properties.

The deposition of active substance during any of these processes may be constant to provide even distribution throughout the coating, or the spraying of the active substance may be varied to result in differing amounts of active substance in the differing polymeric domains within the coating.

In further aspects of the invention, the active substance elution profile is controllable by varying the coating sintering conditions. For example, incomplete sintering will create open spaces, or pores in the interstitial spaces between the polymer particles, which will enable faster eluting of active substance from the coating. Another way to utilize the sintering conditions for elution control would be to deliberately create irregular coatings by foaming during the sintering process. Rapid pressure release of a CO₂- or isobutylene-impregnated polymer film induces formation of foamed polymers which would create a coating with increased porosity and be very 'open' to diffusion/elution. Thus the elution profile would be controllable by manipulating the foaming conditions, which in turn controls the pore density and size.

As used herein, the terms "stent", "stent form", and "stent framework" are used interchangeably.

"Substrate" as used herein, refers to any surface upon which it is desirable to deposit a coating comprising a polymer and a pharmaceutical or active biological agent, wherein the coating process does not substantially modify the morphology of the pharmaceutical agent or the activity of the biological agent. Biomedical implants are of particular interest for the present invention; however the present invention is not intended to be restricted to this class of substrates. Those of skill in the art will appreciate alternate substrates that could benefit from the coating process described herein, such as pharmaceutical tablet cores, as part of an assay apparatus or as components in a diagnostic kit (e.g. a test strip).

"Biomedical implant" as used herein refers to any implant for insertion into the body of a human or animal subject, including but not limited to stents (e.g., vascular stents including but not limited to coronary stents and peripheral stents), electrodes, catheters, leads, implantable pacemaker, cardioverter or defibrillator housings, joints, screws, rods, ophthalmic implants, femoral pins, bone plates, grafts, anastomotic devices, perivascular wraps, sutures, staples, shunts for hydrocephalus, dialysis grafts, colostomy bag attachment devices, ear drainage tubes, leads for pace makers and implantable cardioverters and defibrillators, vertebral disks, bone pins, suture anchors, hemostatic barriers, clamps, screws, plates, clips, vascular implants, tissue adhesives and sealants, tissue scaffolds, various types of dressings (e.g., wound dressings), bone substitutes, intraluminal devices, vascular supports, etc.

The implants may be formed from any suitable material, including but not limited to organic polymers (including stable or inert polymers and biodegradable polymers), metals, inorganic materials such as silicon, and composites thereof, including layered structures with a core of one material and one or more coatings of a different material. Substrates made of a conducting material facilitate electrostatic capture. However, the invention contemplates the use of electrostatic capture in conjunction with substrate having low conductivity or which non-conductive. To enhance electrostatic capture when a non-conductive substrate is employed, the substrate is processed while maintaining a strong electrical field in the vicinity of the substrate.

Subjects into which biomedical implants of the invention may be applied or inserted include both human subjects (including male and female subjects and infant, juvenile, adolescent, adult and geriatric subjects) as well as animal subjects (including but not limited to dog, cat, horse, monkey, etc.) for veterinary purposes.

In a preferred embodiment the biomedical implant is an expandable intraluminal vascular graft or stent (e.g., comprising a wire mesh tube) that can be expanded within a blood vessel by an angioplasty balloon associated with a catheter to dilate and expand the lumen of a blood vessel, such as described in US Patent No. 4,733,665 to Palmaz Shaz.

"Drug" as used herein refers to any of a variety of active agents used to to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms).

An "active agent" as used herein can be a pharmaceutical agent, or an active biological agent as further defined herein.

"Pharmaceutical agent" as used herein refers to any of a variety of drugs, therapeutic agents or pharmaceutical compounds that can be used as active agents to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). It is possible that the pharmaceutical agents of the invention may also comprise two or more drugs or pharmaceutical compounds. Pharmaceutical agents, include but are not limited to antirestenotic agents, antidiabetics, analgesics, antiinflammatory agents, antirheumatics, antihypotensive agents, antihypertensive agents, psychoactive drugs, tranquillizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals, chemotherapeutic agents and amino acids. Examples of suitable active ingredients are acarbose, acetylsalicylic acid, acyclovir, allopurinol, alprostadil, prostaglandins, amantadine, ambroxol, amlodipine, S-aminosalicylic acid, amitriptyline, atenolol, azathioprine, balsalazide, beclomethasone, betahistine, bezafibrate, diazepam and diazepam derivatives, budesonide, bufexamac, buprenorphine, methadone, calcium salts, potassium salts, magnesium salts, candesartan, carbamazepine, captopril, cetirizine, chenodeoxycholic acid, theophylline and theophylline derivatives, trypsins, cimetidine, clobutinol, clonidine, cotrimoxazole, codeine, caffeine, vitamin D and derivatives of vitamin D, colestyramine, cromoglicic acid, coumarin and coumarin derivatives, cysteine, ciclosporin, cyproterone, cytabarine, dapiprazole, desogestrel, desonide, dihydralazine, diltiazem, ergot alkaloids, dimenhydrinate, dimethyl sulphoxide, dimeticone, domperidone and domperidan derivatives, dopamine, doxazosin, doxylamine, benzodiazepines, diclofenac, desipramine, econazole, ACE inhibitors, enalapril, ephedrine, epinephrine, epoetin and epoetin derivatives, morphinans, calcium antagonists, modafinil, orlistat, peptide antibiotics, phenytoin, riluzoles, risedronate, sildenafil, topiramate, estrogen, progestogen and progestogen derivatives, testosterone derivatives, androgen and androgen derivatives, ethenzamide, etofenamate, etofibrate, fenofibrate, etofylline, famciclovir, famotidine, felodipine, fentanyl, fenticonazole, gyrase inhibitors, fluconazole, fluarizine, fluoxetine, flurbiprofen, ibuprofen, fluvastatin, follitropin, formoterol, fosfomicin, furosemide, fusidic acid, gallopamil, ganciclovir, gemfibrozil, ginkgo, Saint John's wort, glibenclamide, urea derivatives as oral antidiabetics, glucagon, glucosamine and glucosamine derivatives, glutathione, glycerol and glycerol derivatives, hypothalamus hormones, guanethidine, halofantrine, haloperidol, heparin (and derivatives), hyaluronic acid, hydralazine, hydrochlorothiazide (and derivatives), salicylates, hydroxyzine, imipramine, indometacin, indoramine, insulin, iodine and iodine derivatives, isoconazole, isoprenaline, glucitol and glucitol derivatives, itraconazole, ketoprofen, ketotifen, lacidipine, lansoprazole, levodopa, levomethadone, thyroid hormones, lipoic acid (and derivatives), lisinopril, lisuride, lofepramine, loperamide, loratadine, maprotiline, mebendazole, mebeverine, meclozine, mefenamic acid, mefloquine, meloxicam, mepindolol, meprobamate, mesalazine, mesuximide, metamizole, metformin, methylphenidate, metixene, metoprolol, metronidazole, mianserin, miconazole, minoxidil, misoprostol, mizolastine, moexipril, morphine and morphine derivatives, evening primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline and adrenaline derivatives, novamine sulfone, noscapine, nystatin, olanzapine, olsalazine, omeprazole, omoconazole, oxaceprol, oxiconazole, oxymetazoline, pantoprazole, paracetamol (acetaminophen), paroxetine, penciclovir, pentazocine, pentifylline, pentoxifylline, perphenazine, pethidine, plant extracts, phenazone, pheniramine, barbituric acid derivatives, phenylbutazone, pimozide, pindolol, piperazine, piracetam, pirenzepine, piribedil, piroxicam, pramipexole, pravastatin, prazosin, procaine, promazine, propiverine, propranolol, propyphenazone, protionamide, proxyphylline, quetiapine, quinapril, quinaprilat, ramipril, ranitidine, reproterol, reserpine, ribavirin, risperidone, ritonavir, ropinirole, roxatidine, ruscogenin, rutoside (and derivatives), sabadilla, salbutamol, salmeterol, scopolamine, selegiline, sertaconazole, sertindole, sertralion, silicates, simvastatin, sitosterol, sotalol, spaglumic acid, spirapril, spironolactone, stavudine, streptomycin, sucralfate, sufentanil, sulfasalazine, sulpiride, sultiam, sumatriptan, suxamethonium chloride, tacrine, tacrolimus, taliolol, taurolidine, temazepam, tenoxicam, terazosin, terbinafine, terbutaline, terfenadine, terlipressin, tertatolol, teryzoline, theobromine, butizine, thiamazole, phenothiazines, tiagabine, tiapride, propionic acid derivatives, ticlopidine, timolol, tinidazole, tioconazole, tioguanine, tioxolone, tiropramide, tizanidine, tolazoline, tolbutamide, tolcapone, tolnaftate, tolperisone, topotecan, torasemide, tramadol, tramazoline, trandolapril, tranylcypromine, trapidil, trazodone, triamcinolone derivatives, triamterene, trifluperidol, trifluridine, trimipramine, tripelennamine, triprolidine, trifosfamide, tromantadine, trometamol, tropalpin, troxerutine, tulobuterol, tyramine, tyrothricin, urapidil, valaciclovir, valproic acid, vancomycin, vecuronium chloride, Viagra, venlafaxine, verapamil, vidarabine, vigabatrin, viloazine, vincamine, vinpocetine, viquidil, warfarin, xantinol nicotinate, xipamide, zafirlukast, zalcitabine, zidovudine, zolmitriptan, zolpidem, zoplicone, zotipine, amphotericin B, caspofungin, voriconazole, resveratrol, PARP-1 inhibitors (including imidazoquinolinone, imidazpyridine, and isoquinolindione, tissue plasminogen activator (tPA), melagatran, lanoteplase, reteplase, staphylokinase, streptokinase, tenecteplase, urokinase, abciximab (ReoPro), eptifibatide, tirofiban, prasugrel, clopidogrel, dipyridamole, cilostazol, VEGF, heparan sulfate, chondroitin sulfate, elongated "RGD" peptide binding domain, CD34 antibodies, cerivastatin, etorvastatin, losartan, valartan, erythropoietin, rosiglitazone, pioglitazone, mutant protein Apo A1 Milano, adiponectin, (NOS) gene therapy, glucagon-like peptide 1, atorvastatin, and atrial natriuretic peptide (ANP), lidocaine, tetracaine, dibucaine, hyssop, ginger, turmeric, Arnica montana, helenalin, cannabichromene, rofecoxib, hyaluronidase, and salts, derivatives, isomers, racemates, diastereoisomers, prodrugs, hydrate, ester, or analogs thereof.

See, e.g., US Patent No. 6,897,205; see also US Patent No. 6,838,528; US Patent No. 6,497,729.

Examples of therapeutic agents employed in conjunction with the invention include, rapamycin, biolimus (biolimus A9), 40-0-(2-Hydroxyethyl)rapamycin (everolimus), 40-0-Benzyl-rapamycin, 40-0-(4'-Hydroxymethyl)benzyl-rapamycin, 40-O-[4'-(1,2-Dihydroxyethyl)]benzyl-rapamycin, 40-0-Allyl-rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin 40-O-(2-Hydroxy)ethoxycar-bonylmethyl-rapamycin, 40-O-(3-Hydroxy)propyl-rapamycin 4O-O-(6-Hydroxy)hexyl-rapamycin 40-O-[2-(2-Hydroxy)ethoxy]ethyl-rapamycin 4O-O-[(3S)-2,2-Dimethyldioxolan-3-yl]methyl-rapamycin, 40-O-[(2S)-2,3-Dihydroxyprop-1-yl]-rapamycin, 4O-O-(2-Acetoxy)ethyl-rapamycin 4O-O-(2-Nicotinoyloxy)ethyl-rapamycin, 4O-O-[2-(N-Morpholino)acetoxy]ethyl-rapamycin 4O-O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methyl-rapamycin, 4O-O-(2-Aminoethyl)-rapamycin, 4O-O-(2-Acetaminoethyl)-rapamycin 4O-O-(2-Nicotinamidoethyl)-rapamycin, 4O-O-(2-(N-Methyl-imidazo-2'-ylcarbethoxamido)ethyl)-rapamycin, 4O-O-(2-Ethoxycarbonylaminoethyl)-rapamycin, 40-O-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), and 42-[3-hydroxy-2-(hydroxymethyl)-2-methylpropanoate]rapamycin (temsirolimus), (42S)-42-Deoxy-42-(1H-tetrazol-1-yl)-rapamycin (zotarolimus), and salts, derivatives, isomers, racemates, diastereoisomers, prodrugs, hydrate, ester, or analogs thereof.

The active ingredients may, if desired, also be used in the form of their pharmaceutically acceptable salts or derivatives (meaning salts which retain the biological effectiveness and properties of the compounds of this invention and which are not biologically or otherwise undesirable), and in the case of chiral active ingredients it is possible to employ both optically active isomers and racemates or mixtures of diastereoisomers.

"Stability" as used herein in refers to the stability of the drug in a polymer coating deposited on a substrate in its final product form (e.g., stability of the drug in a coated stent). The term stability will define 5% or less degradation of the drug in the final product form.

"Active biological agent" as used herein refers to a substance, originally produced by living organisms, that can be used to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). It is possible that the active biological agents of the invention may also comprise two or more active biological agents or an active biological agent combined with a pharmaceutical agent, a stabilizing agent or chemical or biological entity. Although the active biological agent may have been originally produced by living organisms, those of the present invention may also have been synthetically prepared, or by methods combining biological isolation and synthetic modification. By way of a non-limiting example, a nucleic acid could be isolated form from a biological source, or prepared by traditional techniques, known to those skilled in the art of nucleic acid synthesis. Furthermore, the nucleic acid may be further modified to contain non-naturally occurring moieties. Non-limiting examples of active biological agents include peptides, proteins, enzymes, glycoproteins, nucleic acids (including deoxyribonucleotide or ribonucleotide polymers in either single or double stranded form, and unless otherwise limited, encompasses known analogues of natural nucleotides that hybridize to nucleic acids in a manner similar to naturally occurring nucleotides), antisense nucleic acids, fatty acids, antimicrobials, vitamins, hormones, steroids, lipids, polysaccharides, carbohydrates and the like. They further include, but are not limited to, antirestenotic agents, antidiabetics, analgesics, antiinflammatory agents, antirheumatics, antihypotensive agents, antihypertensive agents, psychoactive drugs, tranquillizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals and chemotherapeutic agents. Preferably, the active biological agent is a peptide, protein or enzyme, including derivatives and analogs of natural peptides, proteins and enzymes.

"Activity" as used herein refers to the ability of a pharmaceutical or active biological agent to prevent or treat a disease (meaning any treatment of a disease in a mammal, including preventing the disease, i.e. causing the clinical symptoms of the disease not to develop; inhibiting the disease, i.e. arresting the development of clinical symptoms; and/or relieving the disease, i.e. causing the regression of clinical symptoms). Thus the activity of a pharmaceutical or active biological agent should be of therapeutic or prophylactic value.

"Secondary, tertiary and quaternary structure " as used herein are defined as follows. The active biological agents of the present invention will typically possess some degree of secondary, tertiary and/or quaternary structure, upon which the activity of the agent depends. As an illustrative, non-limiting example, proteins possess secondary, tertiary and quaternary structure. Secondary structure refers to the spatial arrangement of amino acid residues that are near one another in the linear sequence. The α-helix and the β-strand are elements of secondary structure. Tertiary structure refers to the spatial arrangement of amino acid residues that are far apart in the linear sequence and to the pattern of disulfide bonds. Proteins containing more than one polypeptide chain exhibit an additional level of structural organization. Each polypeptide chain in such a protein is called a subunit. Quaternary structure refers to the spatial arrangement of subunits and the nature of their contacts. For example hemoglobin consists of two α and two β chains. It is well known that protein function arises from its conformation or three dimensional arrangement of atoms (a stretched out polypeptide chain is devoid of activity). Thus one aspect of the present invention is to manipulate active biological agents, while being careful to maintain their conformation, so as not to lose their therapeutic activity.

"Polymer" as used herein, refers to a series of repeating monomeric units that have been cross-linked or polymerized. Any suitable polymer can be used to carry out the present invention. It is possible that the polymers of the invention may also comprise two, three, four or more different polymers. In some embodiments, of the invention only one polymer is used. In some preferred embodiments a combination of two polymers are used. Combinations of polymers can be in varying ratios, to provide coatings with differing properties. Those of skill in the art of polymer chemistry will be familiar with the different properties of polymeric compounds. Examples of ploymers that may be used in the present invention include, but are not limited to polycarboxylic acids, cellulosic polymers, , proteins, polypeptides, polyvinylpyrrolidone, maleic anhydride polymers, polyamides, polyvinyl alcohols, polyethylene oxides, glycosaminoglycans, polysaccharides, polyesters, polyurethanes, polystyrenes, copolymers, silicones, polyorthoesters, polyanhydrides, copolymers of vinyl monomers, polycarbonates, polyethylenes, polypropylenes, polylactic acids, polyglycolic acids, polycaprolactones, polyhydroxybutyrate valerates, polyacrylamides, polyethers, polyurethane dispersions, polyacrylates, acrylic latex dispersions, polyacrylic acid, mixtures and copolymers thereof. The polymers of the present invention may be natural or synthetic in origin, including gelatin, chitosan, dextrin, cyclodextrin, Poly(urethanes), Poly(siloxanes) or silicones, Poly(acrylates) such as poly(methyl methacrylate), poly(butyl methacrylate), and Poly(2-hydroxy ethyl methacrylate), Poly(vinyl alcohol) Poly(olefins) such as poly(ethylene), poly(isoprene), halogenated polymers such as Poly(tetrafluoroethylene) - and derivatives and copolymers such as those commonly sold as Teflon® products, Poly(vinylidine fluoride), Poly(vinyl acetate), Poly(vinyl pyrrolidone),. Poly(acrylic acid), Polyacrylamide, Poly(ethylene-co-vinyl acetate), Poly(ethylene glycol), Poly(propylene glycol), Poly(methacrylic acid); etc. Suitable polymers also include absorbable and/or resorbable polymers including the following, combinations, copolymers and derivatives of the following: Polylactides (PLA), Polyglycolides (PGA), Poly(lactide-co-glycolides) (PLGA), Polyanhydrides, Polyorthoesters, Poly(N-(2-hydroxypropyl) methacrylamide), Poly(1-aspartamide), etc.

"Therapeutically desirable morphology" as used herein refers to the gross form and structure of the pharmaceutical agent, once deposited on the substrate, so as to provide for optimal conditions of ex vivo storage, in vivo preservation and/or in vivo release. Such optimal conditions may include, but are not limited to increased shelf life, increased in vivo stability, good biocompatibility, good bioavailability or modified release rates. Typically, for the present invention, the desired morphology of a pharmaceutical agent would be crystalline or semi-crystalline or amorphous, although this may vary widely depending on many factors including, but not limited to, the nature of the pharmaceutical agent, the disease to be treated/prevented, the intended storage conditions for the substrate prior to use or the location within the body of any biomedical implant. Preferably at least 10%, 20%, 30%, 40%, 50%, 60%, 70%, 80%, 90% or 100% of the pharmaceutical agent is in crystalline or semi-crystalline form.

"Stabilizing agent" as used herein refers to any substance that maintains or enhances the stability of the biological agent. Ideally these stabilizing agents are classified as Generally Regarded As Safe (GRAS) materials by the US Food and Drug Administration (FDA). Examples of stabilizing agents include, but are not limited to carrier proteins, such as albumin, gelatin, metals or inorganic salts. Pharmaceutically acceptable excipient that may be present can further be found in the relevant literature, for example in the Handbook of Pharmaceutical Additives: An International Guide to More Than 6000 Products by Trade Name, Chemical, Function, and Manufacturer; Michael and Irene Ash (Eds.); Gower Publishing Ltd.; Aldershot, Hampshire, England, 1995.

"Compressed fluid" as used herein refers to a fluid of appreciable density (e.g., >0.2 g/cc) that is a gas at standard temperature and pressure. "Supercritical fluid", "near-critical fluid", "near-supercritical fluid", "critical fluid", "densified fluid" or "densified gas" as used herein refers to a compressed fluid under conditions wherein the temperature is at least 80% of the critical temperature of the fluid and the pressure is at least 50% of the critical pressure of the fluid.

Examples of substances that demonstrate supercritical or near critical behavior suitable for the present invention include, but are not limited to carbon dioxide, isobutylene, ammonia, water, methanol, ethanol, ethane, propane, butane, pentane, dimethyl ether, xenon, sulfur hexafluoride, halogenated and partially halogenated materials such as chlorofluorocarbons, hydrochlorofluorocarbons, hydrofluorocarbons, perfluorocarbons (such as perfluoromethane and perfuoropropane, chloroform, trichloro-fluoromethane, dichlorodifluoromethane, dichloro-tetrafluoroethane) and mixtures thereof.

"Sintering" as used herein refers to the process by which parts of the matrix or the entire polymer matrix becomes continuous (e.g., formation of a continuous polymer film). As discussed below, the sintering process is controlled to produce a fully conformal continuous matrix (complete sintering) or to produce regions or domains of continuous coating while producing voids (discontinuities) in the matrix. As well, the sintering process is controlled such that some phase separation is obtained between polymer different polymers (e.g., polymers A and B) and/or to produce phase separation between discrete polymer particles. Through the sintering process, the adhesions properties of the coating are improved to reduce flaking of detachment of the coating from the substrate during manipulation in use. As described below, in some embodiments, the sintering process is controlled to provide incomplete sintering of the polymer matrix. In embodiments involving incomplete sintering, a polymer matrix is formed with continuous domains, and voids, gaps, cavities, pores, channels or, interstices that provide space for sequestering a therapeutic agent which is released under controlled conditions. Depending on the nature of the polymer, the size of polymer particles and/or other polymer properties, a compressed gas, a densified gas, a near critical fluid or a super-critical fluid may be employed. In one example, carbon dioxide is used to treat a substrate that has been coated with a polymer and a drug, using dry powder and RESS electrostatic coating processes. In another example, isobutylene is employed in the sintering process. In other examples a mixture of carbon dioxide and isobutylene is employed.

When an amorphous material is heated to a temperature above its glass transition temperature, or when a crystalline material is heated to a temperature above a phase transition temperature, the molecules comprising the material are more mobile, which in turn means that they are more active and thus more prone to reactions such as oxidation. However, when an amorphous material is maintained at a temperature below its glass transition temperature, its molecules are substantially immobilized and thus less prone to reactions. Likewise, when a crystalline material is maintained at a temperature below its phase transition temperature, its molecules are substantially immobilized and thus less prone to reactions. Accordingly, processing drug components at mild conditions, such as the deposition and sintering conditions described herein, minimizes cross-reactions and degradation of the drug component. One type of reaction that is minimized by the processes of the invention relates to the ability to avoid conventional solvents which in turn minimizes autoxidation of drug, whether in amorphous, semi-crystalline, or crystalline form, by reducing exposure thereof to free radicals, residual solvents and autoxidation initiators.

"Rapid Expansion of Supercritical Solutions" or "RESS" as used herein involves the dissolution of a polymer into a compressed fluid, typically a supercritical fluid, followed by rapid expansion into a chamber at lower pressure, typically near atmospheric conditions . The rapid expansion of the supercritical fluid solution through a small opening, with its accompanying decrease in density, reduces the dissolution capacity of the fluid and results in the nucleation and growth of polymer particles. The atmosphere of the chamber is maintained in an electrically neutral state by maintaining an isolating "cloud" of gas in the chamber. Carbon dioxide or other appropriate gas is employed to prevent electrical charge is transferred from the substrate to the surrounding environment.

"Bulk properties" properties of a coating including a pharmaceutical or a biological agent that can be enhanced through the methods of the invention include for example: adhesion, smoothness, conformality, thickness, and compositional mixing.

"Solution Enhanced Dispersion of Supercritical Solutions" or "SEDS" as used herein involves a spray process for the generation of polymer particles, which are formed when a compressed fluid (e.g. supercritical fluid, preferably supercritical CO₂) is used as a diluent to a vehicle in which a polymer dissolved, (one that can dissolve both the polymer and the compressed gas). The mixing of the compressed fluid diluent with the polymer-containing solution may be achieved by encounter of a first stream containing the polymer solution and a second stream containing the diluent compressed fluid, for example, within one co-axial spray nozzle or by the use of multiple spray nozzles or by the use of multiple fluid streams co-entering into a mixing zone. The solvent in the polymer solution may be one compound or a mixture of two or more ingredients and may be or comprise an alcohol (including diols, triols, etc.), ether, amine, ketone, carbonate, or alkanes, or hydrocarbon (aliphatic or aromatic) or may be a mixture of compounds, such as mixtures of alkanes, or mixtures of one or more alkanes in combination with additional compounds such as one or more alcohols, (e.g., from 0 or 0.1 to 5% of a C₁ to C₁₅ alcohol, including diols, triols, etc.). See for example US Patent No. 6,669,785. The solvent may optionally contain a surfactant, as also described in (for example) US Patent No. 6,669,785.

In one embodiment of the SEDS process, a first stream of fluid comprising a polymer dissolved in a common solvent is co-sprayed with a second stream of compressed fluid. Polymer particles are produced as the second stream acts as a diluent that weakens the solvent in the polymer solution of the first stream. The now combined streams of fluid, along with the polymer particles, flow into a collection vessel. In another embodiment of the SEDS process, a first stream of fluid comprising a drug dissolved in a common solvent is co-sprayed with a second stream of compressed fluid. Drug particles are produced as the second stream acts as a diluent that weakens the solvent in the drug solution of the first stream. The now combined streams of fluid, along with the drug particles, flow out into a collection vessel. Control of particle size, particle size distribution, and morphology is achieved by tailoring the following process variables: temperature, pressure, solvent composition of the first stream, flow-rate of the first stream, flow-rate of the second stream, composition of the second stream (where soluble additives may be added to the compressed gas), and conditions of the capture vessel. Typically the capture vessel contains a fluid phase that is at least five to ten times (5-10x) atmospheric pressure.

"Electrostatically charged" or "electrical potential" or "electrostatic capture" as used herein refers to the collection of the spray-produced particles upon a substrate that has a different electrostatic potential than the sprayed particles. Thus, the substrate is at an attractive electronic potential with respect to the particles exiting, which results in the capture of the particles upon the substrate, i.e. the substrate and particles are oppositely charged, and the particles transport through the fluid medium of the capture vessel onto the surface of the substrate is enhanced via electrostatic attraction. This may be achieved by charging the particles and grounding the substrate or conversely charging the substrate and grounding the particles, or by some other process, which would be easily envisaged by one of skill in the art of electrostatic capture.

"Open vessel" as used herein refers to a vessel open to the outside atmosphere, and thus at substantially the same temperature and pressure as the outside atmosphere.

"Closed vessel" as used herein refers to a vessel sealed from the outside atmosphere, and thus may be at significantly different temperatures and pressures to the outside atmosphere.

Means for creating the bioabsorbable polymer(s) + drug (s) matrix on the stent-form - forming the final device:
- Spray coat the stent-form with drug and polymer as is done in Micell process (e-RESS, e-DPC, compressed-gas sintering).
- Perform multiple and sequential coating-sintering steps where different materials may be deposited in each step, thus creating a laminated structure with a multitude of thin layers of drug(s), polymer(s) or drug+polymer that build the final stent.
- Perform the deposition of polymer(s) + drug(s) laminates with the inclusion of a mask on the inner (luminal) surface of the stent. Such a mask could be as simple as a non-conductive mandrel inserted through the internal diameter of the stent form. This masking could take place prior to any layers being added, or be purposefully inserted after several layers are deposited continuously around the entire stent-form.

Another advantage of the present invention is the ability to create a stent with a controlled (dialed-in) drug-elution profile. Via the ability to have different materials in each layer of the laminate structure and the ability to control the location of drug(s) independently in these layers, the method enables a stent that could release drugs at very specific elution profiles, programmed sequential and/or parallel elution profiles. Also, the present invention allows controlled elution of one drug without affecting the elution of a second drug (or different doses of the same drug).

The embodiments incorporating a stent form or framework provide the ability to radiographically monitor the stent in deployment. In an alternative embodiment, the inner-diameter of the stent can be masked (e.g. by a non-conductive mandrel). Such masking would prevent additional layers from being on the interior diameter (abluminal) surface of the stent. The resulting configuration may be desirable to provide preferential elution of the drug toward the vessel wall (luminal surface of the stent) where the therapeutic effect of anti-restenosis is desired, without providing the same antiproliferative drug(s) on the abluminal surface, where they may retard healing, which in turn is suspected to be a cause of late-stage safety problems with current DESs.

The present invention provides numerous advantages. The invention is advantageous allows for employing a platform combining layer formation methods based on compressed fluid technologies; electrostatic capture and sintering methods. The platform results in drug eluting stents having enhanced therapeutic and mechanical properties. The invention is particularly advantageous in that it employs optimized laminate polymer technology. In particular, the present invention allows the formation of discrete layers of specific drug platforms.

Conventional processes for spray coating stents require that drug and polymer be dissolved in solvent or mutual solvent before spray coating can occur. The platform provided herein the drugs and polymers are coated on the stent framework in discrete steps, which can be carried out simultaneously or alternately. This allows discrete deposition of the active agent (e.g.; a drug) within a polymer matrix thereby allowing the placement of more than one drug on a single medical device with or without an intervening polymer layer. For example, the present platform provides a dual drug eluting stent.

Some of the advantages provided by the subject invention include employing compressed fluids (e.g., supercritical fluids, for example E-RESS based methods); solvent free deposition methodology; a platform that allows processing at lower temperatures thereby preserving the qualities of the active agent and the polymer matrix; the ability to incorporate two, three or more drugs while minimizing deleterious effects from direct interactions between the various drugs and/or their excipients during the fabrication and/or storage of the drug eluting stents; a dry deposition; enhanced adhesion and mechanical properties of the layers on the stent framework; precision deposition and rapid batch processing; and ability to form intricate structures.

In one embodiment, the present invention provides a multi-drug delivery platform which produces strong, resilient and flexible drug eluting stents including an anti-restenosis drug (e.g.; a limus or taxol) and anti-thrombosis drug (e.g.; heparin or an analog thereof) and well characterized bioabsorbable polymers. The drug eluting stents provided herein minimize potential for thrombosis, in part, by reducing or totally eliminating thrombogenic polymers and reducing or totally eliminating residual drugs that could inhibit healing.

The platform provides optimized delivery of multiple drug therapies for example for early stage treatment (restenosis) and late-stage (thrombosis).

The platform also provides an adherent coating which enables access through tortuous lesions without the risk of the coating being compromised.

Another advantage of the present platform is the ability to provide highly desirable eluting profiles

Advantages of the invention include the ability to reduce or completely eliminate potentially thrombogenic polymers as well as possibly residual drugs that may inhibit long term healing. As well, the invention provides advantageous stents having optimized strength and resilience if coatings which in turn allows access to complex lesions and reduces or completely eliminates delamination. Laminated layers of bioabsorbable polymers allow controlled elution of one or more drugs.

The platform provided herein reduces or completely eliminates shortcoming that have been associated with conventional drug eluting stents. For example, the platform provided herein allows for much better tuning of the period of time for the active agent to elute and the period of time necessary for the polymer matrix to resorb thereby minimizing thrombosis and other deleterious effects associate with poorly controlled drug release.

The present invention provides several advantages which overcome or attenuate the limitations of current technology for bioabsorbable stents. For example, an inherent limitation of conventional bioabsorbable polymeric materials relates to the difficulty in forming to a strong, flexible, deformable (e.g. balloon deployable) stent with low profile. The polymers generally lack the strength of high-performance metals. The present invention overcomes these limitations by creating a laminate structure in the essentially polymeric stent. Without wishing to be bound by any specific theory or analogy, the increased strength provided by the stents of the invention can be understood by comparing the strength of plywood vs. the strength of a thin sheet of wood.

Embodiments of the invention involving a thin metallic stent-framework provide advantages including the ability to overcome the inherent elasticity of most polymers. It is generally difficult to obtain a high rate (e.g., 100%) of plastic deformation in polymers (compared to elastic deformation where the materials have some 'spring back' to the original shape). Again, without wishing to be bound by any theory, the central metal stent framework (that would be too small and weak to serve as a stent itself) would act like wires inside of a plastic, deformable stent, basically overcoming any 'elastic memory' of the polymer.

Provided herein is a composition comprising poly(D,L-lactic-co-glycolic acid) substantially free of acidic impurities.

In some embodiments, the poly(D,L-lactic-co-glycolic acid) contains less than 0.5 % (wt/wt) of acidic impurity. In some embodiments, the poly(D,L-lactic-co-glycolic acid) contains less than 1.0 % (wt/wt) of acidic impurity. In some embodiments, the poly(D,L-lactic-co-glycolic acid) contains less than 1.5 % (wt/wt) of acidic impurity. In some embodiments, the poly(D,L-lactic-co-glycolic acid) has a ratio of lactic acid monomer to glycolic acid monomer ranging from 82:18 to 88:12. In some embodiments, the poly(D,L-lactic-co-glycolic acid) has a ratio of lactic acid monomer to glycolic acid monomer ranging from 72:28 to 78:22. In some embodiments, the poly(D,L-lactic-co-glycolic acid) has a ratio of lactic acid monomer to glycolic acid monomer ranging from 62:38 to 68:32. In some embodiments, the poly(D,L-lactic-co-glycolic acid) has a ratio of lactic acid monomer to glycolic acid monomer ranging from 47:53 to 53:47.

In some embodiments, the poly(D,L-lactic-co-glycolic acid) has a weight average molecular weight of about 4,000 to about 8,000. In some embodiments, the poly(D,L-lactic-co-glycolic acid) has a weight average molecular weight of about 8,000 to about 12,000. In some embodiments, the poly(D,L-lactic-co-glycolic acid) has a weight average molecular weight of about 12,000 to about 16,000. In some embodiments, the poly(D,L-lactic-co-glycolic acid) has a weight average molecular weight of up to about 90 kDalton.

Provided herein is a method for the preparation of a composition comprising poly(D,L-lactic-co-glycolic acid) substantially free of acidic impurities, said method comprising: contacting poly(D,L-lactic-co-glycolic acid) containing acidic impurties with a solid base; forming a salt of the acidic impurity; and separating the poly(D,L-lactic-co-glycolic acid) from the salt of the acidic impurity.

In some embodiments, the solid base is selected from the group consisting of: MgO, LiH, NaH, KH, MgH₂, and CaH₂. In some embodiments, the solid base is CaH₂.

Provided herein is a method for the preparation of a composition comprising poly(D,L-lactic-co-glycolic acid) substantially free of acidic impurities, said method comprising: dissolving the poly(D,L-lactic-co-glycolic acid) containing acidic impurties in an inert solvent; contacting poly(D,L-lactic-co-glycolic acid) solution with a metal hydride; forming a metal salt of the acidic impurity; and separating the poly(D,L-lactic-co-glycolic acid) from the metal salt of the acidic impurity.

In some embodiments, the solid base is selected from the group consisting of: MgO, LiH, LiAlH₄, NaH, NaBH₄, KH, MgH₂, and CaH₂. In some embodiments, the solid base is CaH₂. In some embodiments, the metal salt of the acidic impurity is separated from the poly(D,L-lactic-co-glycolic acid) by filtration. In some embodiments, the inert solvent is an organic solvent. In some embodiments, the salt of the acidic impurity is separated from the poly(D,L-lactic-co-glycolic acid) by diffusion through a semi-permeable membrane.

In some embodiments, the method is performed in a supercritical state.

In some embodiments, the inert solvent is a fluorocarbon. In some embodiments, the fluorocarbon solvent is FC236.

Provided herein is a method for the preparation of a composition comprising poly(D,L-lactic-co-glycolic acid) substantially free of acidic impurities, said method comprising: forming the poly(D,L-lactic-co-glycolic acid) containing acidic impurties into a thin film; contacting said poly(D,L-lactic-co-glycolic acid) thin film with a layer of solid base; diffusing the acidic impurties from said poly(D,L-lactic-co-glycolic acid) thin film; and separating the poly(D,L-lactic-co-glycolic acid) thin film from the layer of solid base.

In some embodiments, the solid base is selected from the group consisting of: MgO, LiH, NaH, KH, MgH2, and CaH₂. In some embodiments, the solid base is CaH₂.

Provided herein is a method for the preparation of a composition comprising poly(D,L-lactic-co-glycolic acid) substantially free of acidic impurities, said method comprising subjecting the poly(D,L-lactic-co-glycolic acid) containing acidic impurties to electrophoresis.

Provided herein is a device comprising: a substrate, and a coating wherein the coating comprises poly(D,L-lactic-co-glycolic acid) substantially free of acidic impurities.

Provided herein is a device comprising: a substrate, and a coating wherein the coating comprises the composition comprising poly(D,L-lactic-co-glycolic acid) substantially free of acidic impurities.

Provided herein is a device comprising: a substrate, and a coating wherein the coating comprises the composition comprising poly(D,L-lactic-co-glycolic acid) substantially free of acidic impurities, formed by any of the methods described herein.

In some embodiments, the substrate comprises a stent framework. In some embodiments, the substrate is a biomedical implant selected from the group consisting of stents (e.g., vascular stents), electrodes, catheters, leads, implantable pacemaker, cardioverter or defibrillator housings, joints, screws, rods, ophthalmic implants, femoral pins, bone plates, grafts, anastomotic devices, perivascular wraps, sutures, staples, shunts for hydrocephalus, dialysis grafts, colostomy bag attachment devices, ear drainage tubes, leads for pace makers and implantable cardioverters and defibrillators, vertebral disks, bone pins, suture anchors, hemostatic barriers, clamps, screws, plates, clips, vascular implants, tissue adhesives and sealants, tissue scaffolds, various types of dressings (e.g., wound dressings), bone substitutes, intraluminal devices, and vascular supports.

In some embodiments, the coating comprises rapamycin wherein at least part of rapamycin is in crystalline form.

In some embodiments, the coating has substantially uniform thickness and rapamycin in the coating is substantially uniformly dispersed.

In some embodiments, the average rapamycin content varies along the length of said device.

In some embodiments, at least part of said rapamycin forms a phase separate from one or more phases formed by said poly(D,L-lactic-co-glycolic acid) .

In some embodiments, the rapamycin is at least 50% crystalline. In some embodiments, the rapamycin is at least 75% crystalline. In some embodiments, the rapamycin is at least 90% crystalline. In some embodiments, the rapamycin is at least 95% crystalline. In some embodiments, the rapamycin is at least 99% crystalline.

In some embodiments, the polymer is a mixture of two or more polymers, wherein at least one of the polymers is said poly(D,L-lactic-co-glycolic acid) . In some embodiments, the mixture of polymers forms a continuous film around particles of rapamycin. In some embodiments, two or more polymers are intimately mixed. In some embodiments, the mixture comprises no single polymer domain larger than about 20 nm. In some embodiments, each polymer in said mixture comprises a discrete phase . In some embodiments, the discrete phases formed by said polymers in said mixture are larger than about 10nm. In some embodiments, the discrete phases formed by said polymers in said mixture are larger than about 50nm.

In some embodiments, the rapamycin in said device has a shelf stability of at least 3 months. In some embodiments, the rapamycin in said device has a shelf stability of at least 6 months. In some embodiments, the rapamycin in said device has a shelf stability of at least 12 months. In some embodiments, the device provides an elution profile wherein about 10% to about 50% of rapamycin is eluted at week 1 after the composite is implanted in a subject under physiological conditions, about 25% to about 75% of rapamycin is eluted at week 2 and about 50% to about 100% of rapamycin is eluted at week 4.

In some embodiments, the coating comprises a macrolide immunosuppressive (limus) drug-polymer coating wherein at least part of the drug is in crystalline form. In some embodiments, the macrolide immunosuppressive drug comprises one or more of rapamycin, biolimus (biolimus A9), 40-0-(2-Hydroxyethyl)rapamycin (everolimus), 40-0-Benzyl-rapamycin, 40-0-(4'-Hydroxymethyl)benzyl-rapamycin, 40-O-[4'-(1,2-Dihydroxyethyl)]benzyl-rapamycin, 40-0-Allyl-rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin 40-O-(2-Hydroxy)ethoxycar-bonylmethyl-rapamycin, 40-O-(3-Hydroxy)propyl-rapamycin 4O-O-(6-Hydroxy)hexyl-rapamycin 40-O-[2-(2-Hydroxy)ethoxy]ethyl-rapamycin 4O-O-[(3S)-2,2-Dimethyldioxolan-3-yl]methyl-rapamycin, 40-O-[(2S)-2,3-Dihydroxyprop-1-yl]-rapamycin, 4O-O-(2-Acetoxy)ethyl-rapamycin 4O-O-(2-Nicotinoyloxy)ethyl-rapamycin, 4O-O-[2-(N-Morpholino)acetoxy]ethyl-rapamycin 4O-O-(2-N-Imidazolylacetoxy)ethyl-rapamycin, 40-O-[2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methyl-rapamycin, 4O-O-(2-Aminoethyl)-rapamycin, 4O-O-(2-Acetaminoethyl)-rapamycin 4O-O-(2-Nicotinamidoethyl)-rapamycin, 4O-O-(2-(N-Methyl-imidazo-2'-ylcarbethoxamido)ethyl)-rapamycin, 4O-O-(2-Ethoxycarbonylaminoethyl)-rapamycin, 40-O-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), and 42-[3-hydroxy-2-(hydroxymethyl)-2-methylpropanoate]rapamycin (temsirolimus), (42S)-42-Deoxy-42-(1H-tetrazol-1-yl)-rapamycin (zotarolimus), and salts, derivatives, isomers, racemates, diastereoisomers, prodrugs, hydrate, ester, or analogs thereof. In some embodiments, the macrolide immunosuppressive drug is at least 50% crystalline.

In some embodiments, the coating comprises a pharmaceutical agent. In some embodiments, the pharmaceutical agent is selected form the group consisting of antirestenotic agents, antidiabetics, analgesics, antiinflammatory agents, antirheumatics, antihypotensive agents, antihypertensive agents, psychoactive drugs, tranquillizers, antiemetics, muscle relaxants, glucocorticoids, agents for treating ulcerative colitis or Crohn's disease, antiallergics, antibiotics, antiepileptics, anticoagulants, antimycotics, antitussives, arteriosclerosis remedies, diuretics, proteins, peptides, enzymes, enzyme inhibitors, gout remedies, hormones and inhibitors thereof, cardiac glycosides, immunotherapeutic agents and cytokines, laxatives, lipid-lowering agents, migraine remedies, mineral products, otologicals, anti parkinson agents, thyroid therapeutic agents, spasmolytics, platelet aggregation inhibitors, vitamins, cytostatics and metastasis inhibitors, phytopharmaceuticals, chemotherapeutic agents and amino acids. Examples of suitable active ingredients are acarbose, acetylsalicylic acid, acyclovir, allopurinol, alprostadil, prostaglandins, amantadine, ambroxol, amlodipine, S-aminosalicylic acid, amitriptyline, atenolol, azathioprine, balsalazide, beclomethasone, betahistine, bezafibrate, diazepam and diazepam derivatives, budesonide, bufexamac, buprenorphine, methadone, calcium salts, potassium salts, magnesium salts, candesartan, carbamazepine, captopril, cetirizine, chenodeoxycholic acid, theophylline and theophylline derivatives, trypsins, cimetidine, clobutinol, clonidine, cotrimoxazole, codeine, caffeine, vitamin D and derivatives of vitamin D, colestyramine, cromoglicic acid, coumarin and coumarin derivatives, cysteine, ciclosporin, cyproterone, cytabarine, dapiprazole, desogestrel, desonide, dihydralazine, diltiazem, ergot alkaloids, dimenhydrinate, dimethyl sulphoxide, dimeticone, domperidone and domperidan derivatives, dopamine, doxazosin, doxylamine, benzodiazepines, diclofenac, desipramine, econazole, ACE inhibitors, enalapril, ephedrine, epinephrine, epoetin and epoetin derivatives, morphinans, calcium antagonists, modafinil, orlistat, peptide antibiotics, phenytoin, riluzoles, risedronate, sildenafil, topiramate, estrogen, progestogen and progestogen derivatives, testosterone derivatives, androgen and androgen derivatives, ethenzamide, etofenamate, etofibrate, fenofibrate, etofylline, famciclovir, famotidine, felodipine, fentanyl, fenticonazole, gyrase inhibitors, fluconazole, fluarizine, fluoxetine, flurbiprofen, ibuprofen, fluvastatin, follitropin, formoterol, fosfomicin, furosemide, fusidic acid, gallopamil, ganciclovir, gemfibrozil, ginkgo, Saint John's wort, glibenclamide, urea derivatives as oral antidiabetics, glucagon, glucosamine and glucosamine derivatives, glutathione, glycerol and glycerol derivatives, hypothalamus hormones, guanethidine, halofantrine, haloperidol, heparin (and derivatives), hyaluronic acid, hydralazine, hydrochlorothiazide (and derivatives), salicylates, hydroxyzine, imipramine, indometacin, indoramine, insulin, iodine and iodine derivatives, isoconazole, isoprenaline, glucitol and glucitol derivatives, itraconazole, ketoprofen, ketotifen, lacidipine, lansoprazole, levodopa, levomethadone, thyroid hormones, lipoic acid (and derivatives), lisinopril, lisuride, lofepramine, loperamide, loratadine, maprotiline, mebendazole, mebeverine, meclozine, mefenamic acid, mefloquine, meloxicam, mepindolol, meprobamate, mesalazine, mesuximide, metamizole, metformin, methylphenidate, metixene, metoprolol, metronidazole, mianserin, miconazole, minoxidil, misoprostol, mizolastine, moexipril, morphine and morphine derivatives, evening primrose, nalbuphine, naloxone, tilidine, naproxen, narcotine, natamycin, neostigmine, nicergoline, nicethamide, nifedipine, niflumic acid, nimodipine, nimorazole, nimustine, nisoldipine, adrenaline and adrenaline derivatives, novamine sulfone, noscapine, nystatin, olanzapine, olsalazine, omeprazole, omoconazole, oxaceprol, oxiconazole, oxymetazoline, pantoprazole, paracetamol (acetaminophen), paroxetine, penciclovir, pentazocine, pentifylline, pentoxifylline, perphenazine, pethidine, plant extracts, phenazone, pheniramine, barbituric acid derivatives, phenylbutazone, pimozide, pindolol, piperazine, piracetam, pirenzepine, piribedil, piroxicam, pramipexole, pravastatin, prazosin, procaine, promazine, propiverine, propranolol, propyphenazone, protionamide, proxyphylline, quetiapine, quinapril, quinaprilat, ramipril, ranitidine, reproterol, reserpine, ribavirin, risperidone, ritonavir, ropinirole, roxatidine, ruscogenin, rutoside (and derivatives), sabadilla, salbutamol, salmeterol, scopolamine, selegiline, sertaconazole, sertindole, sertralion, silicates, simvastatin, sitosterol, sotalol, spaglumic acid, spirapril, spironolactone, stavudine, streptomycin, sucralfate, sufentanil, sulfasalazine, sulpiride, sultiam, sumatriptan, suxamethonium chloride, tacrine, tacrolimus, taliolol, taurolidine, temazepam, tenoxicam, terazosin, terbinafine, terbutaline, terfenadine, terlipressin, tertatolol, teryzoline, theobromine, butizine, thiamazole, phenothiazines, tiagabine, tiapride, propionic acid derivatives, ticlopidine, timolol, tinidazole, tioconazole, tioguanine, tioxolone, tiropramide, tizanidine, tolazoline, tolbutamide, tolcapone, tolnaftate, tolperisone, topotecan, torasemide, tramadol, tramazoline, trandolapril, tranylcypromine, trapidil, trazodone, triamcinolone derivatives, triamterene, trifluperidol, trifluridine, trimipramine, tripelennamine, triprolidine, trifosfamide, tromantadine, trometamol, tropalpin, troxerutine, tulobuterol, tyramine, tyrothricin, urapidil, valaciclovir, valproic acid, vancomycin, vecuronium chloride, Viagra, venlafaxine, verapamil, vidarabine, vigabatrin, viloazine, vincamine, vinpocetine, viquidil, warfarin, xantinol nicotinate, xipamide, zafirlukast, zalcitabine, zidovudine, zolmitriptan, zolpidem, zoplicone, zotipine, amphotericin B, caspofungin, voriconazole, resveratrol, PARP-1 inhibitors (including imidazoquinolinone, imidazpyridine, and isoquinolindione, tissue plasminogen activator (tPA), melagatran, lanoteplase, reteplase, staphylokinase, streptokinase, tenecteplase, urokinase, abciximab (ReoPro), eptifibatide, tirofiban, prasugrel, clopidogrel, dipyridamole, cilostazol, VEGF, heparan sulfate, chondroitin sulfate, elongated "RGD" peptide binding domain, CD34 antibodies, cerivastatin, etorvastatin, losartan, valartan, erythropoietin, rosiglitazone, pioglitazone, mutant protein Apo A1 Milano, adiponectin, (NOS) gene therapy, glucagon-like peptide 1, atorvastatin, and atrial natriuretic peptide (ANP), lidocaine, tetracaine, dibucaine, hyssop, ginger, turmeric, Arnica montana, helenalin, cannabichromene, rofecoxib, hyaluronidase, and salts, derivatives, isomers, racemates, diastereoisomers, prodrugs, hydrate, ester, or analogs thereof.

In some embodiments, the coating has substantially uniform thickness and covers substantially the entire surface of said substrate.

In some embodiments, the coating comprises a microstructure. In some embodiments, pharmaceutical particles are sequestered or encapsulated within said microstructure. In some embodiments, the microstructure comprises microchannels, micropores and/or microcavities. In some embodiments, the microstructure is selected to allow sustained release of said at least one pharmaceutical agent. In some embodiments, the microstructure is selected to allow controlled release of said at least one pharmaceutical agent.

In some embodiments, the coating comprises at least two pharmaceutical agents. In some embodiments, the pharmaceutical agent is in the form of particles having an average diameter from 2 nm to 500 nm.

Provided herein is a method of depositing a coating onto a substrate, said coating comprising: at least one polymer comprising poly(D,L-lactic-co-glycolic acid) substantially free of acidic impurities; and at least one pharmaceutical agent in a therapeutically desirable morphology and/or at least one active biological agent; said method comprising the following steps: discharging the at least one pharmaceutical agent and/or at least one active biological agent in dry powder form through a first orifice; discharging the at least one polymer in dry powder form through a second orifice; depositing the polymer and pharmaceutical agent and/or active biological agent particles onto said substrate, wherein an electrical potential is maintained between the substrate and the polymer and pharmaceutical agent and/or active biological agent particles, thereby forming said coating; and sintering said coating under conditions that do not substantially modify the morphology of said pharmaceutical agent and/or the activity of said biological agent.

Provided herein is a method of depositing a coating onto a substrate, said coating comprising: at least one polymer comprising poly(D,L-lactic-co-glycolic acid) substantially free of acidic impurities; and at least one pharmaceutical agent in a therapeutically desirable morphology and/or at least one active biological agent;
said method comprising the following steps: discharging the at least one pharmaceutical agent and/or at least one active biological agent in dry powder form through a first orifice; forming a supercritical or near supercritical fluid solution comprising at least one supercritical fluid solvent and at least one polymer and discharging said supercritical or near supercritical fluid solution through a second orifice under conditions sufficient to form solid particles of the polymer; depositing the polymer and pharmaceutical agent and/or active biological agent particles onto said substrate, wherein an electrical potential is maintained between the substrate and the polymer and pharmaceutical agent and/or active biological agent particles, thereby forming said coating; and sintering said coating under conditions that do not substantially modify the morphology of said pharmaceutical agent and/or the activity of said biological agent.

Provided herein is a method of depositing a coating onto a substrate, said coating comprising: at least one polymer comprising poly(D,L-lactic-co-glycolic acid) substantially free of acidic impurities; and at least one pharmaceutical agent in a therapeutically desirable morphology in dry powder form and/or at least one active biological agent; said method comprising the following steps: discharging the at least one pharmaceutical agent and/or at least one active biological agent through a first orifice; forming a first stream of a polymer solution comprising at least one solvent and at least one polymer; forming a second stream of a supercritical or near supercritical fluid comprising at least one supercritical fluid; contacting said first and second streams, whereby said supercritical or near supercritical fluid acts as a diluent of said solution under conditions sufficient to form particles of said polymer; depositing the polymer and pharmaceutical agent and/or active biological agent particles onto said substrate, wherein an electrical potential is maintained between the substrate and the polymer and pharmaceutical agent and/or active biological agent particles, thereby forming said coating; and sintering said coating under conditions that do not substantially modify the morphology of said pharmaceutical agent and/or the activity of said biological agent.

In some embodiments, the at least one polymer comprising poly(D,L-lactic-co-glycolic acid) substantially free of acidic impurities is formed by any of the methods described herein. In some embodiments, the at least one polymer comprising poly(D,L-lactic-co-glycolic acid) substantially free of acidic impurities is one of the compositions described herein.

In some embodiments, the method further comprises depositing a top layer on said coating.

In some embodiments, the top layer is a polymer film.

The method of some embodiments is carried out in an open vessel. The method of some embodiments is carried out in a closed vessel.

In some embodiments, the first and said second orifices are provided as one single orifice.

In some embodiments, the polymer and said pharmaceutical agent and/or active biological agent are mixed together prior to discharging.

In some embodiments, the polymer and said pharmaceutical agent and/or active biological agent particles are discharged simultaneously.

In some embodiments, the polymer and said pharmaceutical agent and/or active biological agent are discharged in succession.

In some embodiments, the first and the second orifices are discharged to form a multilayer coating.

In some embodiments, the pharmaceutical agent and/or active biological agent is evenly dispersed throughout said coating.

In some embodiments, the pharmaceutical agent and/or active biological agent is not evenly dispersed throughout said coating.

The method of some embodiments further comprises discharging a third dry powder comprising a second pharmaceutical agent in a therapeutically desirable morphology in dry powder form and/or active biological agent whereby a coating comprising at least two different pharmaceutical agents and/or active biological agents is deposited on said substrate.

In some embodiments, the substrate is electrostatically charged.

In some embodiments, the substrate is a biomedical implant. In some embodiments, the biomedical implant is selected from the group consisting of stents, joints, screws, rods, pins, plates, staples, shunts, clamps, clips, sutures, suture anchors, electrodes, catheters, leads, grafts, dressings, pacemakers, pacemaker housings, cardioverters, cardioverter housings, defibrillators, defibrillator housings, prostheses, ear drainage tubes, ophthalmic implants, orthopedic devices, vertebral disks, bone substitutes, anastomotic devices, perivascular wraps, colostomy bag attachment devices, hemostatic barriers, vascular implants, vascular supports, tissue adhesives, tissue sealants, tissue scaffolds and intraluminal devices.

In some embodiments, the substrate is biodegradable. In some embodiments, the substrate and said coating are biodegradable.

In some embodiments, the therapeutically desirable morphology of said pharmaceutical agent is crystalline or semi-crystalline.

In some embodiments, at least 50% of said pharmaceutical agent in powder form is crystalline or semicrystalline.

In some embodiments, the pharmaceutical agent comprises at least one drug.

In some embodiments, the at least one drug is selected from the group consisting of antirestenotic agents, antidiabetics, analgesics, antiinflammatory agents, antirheumatics, antihypotensive agents, antihypertensive agents.

In some embodiments, the activity of said active biological agent is of therapeutic or prophylactic value.

In some embodiments, the biological agent is selected from the group comprising peptides, proteins, enzymes, nucleic acids, antisense nucleic acids, antimicrobials, vitamins, hormones, steroids, lipids, polysaccharides and carbohydrates.

In some embodiments, the activity of said active biological agent is influenced by the secondary, tertiary or quaternary structure of said active biological agent.

In some embodiments, the active biological agent possesses a secondary, tertiary or quaternary structure which is not substantially changed after the step of sintering said coating.

In some embodiments, the active biological agent further comprises a stabilizing agent.

In some embodiments, the sintering comprises treating said coated substrate with a compressed gas, compressed liquid or supercritical fluid that is a non-solvent for both the polymer and the pharmaceutical and/or biological agents.

In some embodiments, the compressed gas, compressed liquid or supercritical fluid comprises carbon dioxide, isobutylene or a mixture thereof.

In some embodiments, the at least one polymer comprises two or more polymers, wherein the first polymer swells in aqueous media and the second polymer does not substantially swell in aqueous media.

In some embodiments, in aqueous media the pharmaceutical agent and/or active biological agent elutes from said first polymer, and substantially does not elute from second polymer.

In some embodiments, the elution profile of said pharmaceutical agent and/or active biological agent is controllable by altering at least one parameter selected from the group consisting of the relative polymer amounts, the polymer particle sizes, the polymer particle shapes, the physical distribution of the polymers, the sintering conditions or any combination thereof.

Provided herein is a method for depositing a coating comprising a polymer and pharmaceutical agent on a substrate, wherein the polymer comprises poly(D,L-lactic-co-glycolic acid) substantially free of acidic impurities and wherein the method comprises: forming a supercritical or near critical fluid mixture that includes at least one polymer and at least one pharmaceutical agent discharging a spray of the supercritical or near critical fluid mixture through a constriction under conditions sufficient to form particles of the pharmaceutical agent and particles of the polymer that are substantially free of supercritical fluid solvent or solvents, wherein the constriction comprises an insulator material; providing a first electrode that is secured to the constriction and that can generate an electrical field for charging the solid pharmaceutical particles and/or the polymer particles to a first electric potential after they exit the constriction; depositing the charged solid pharmaceutical particles and polymer particles to form a coating onto said substrate; and sintering said coating under conditions that do not substantially modify the morphology of said solid pharmaceutical particles.

In some embodiments, the poly(D,L-lactic-co-glycolic acid) substantially free of acidic impurities is formed by any of the methods described herein.

In some embodiments, the first electrode is located adjacent the spray discharge from the constriction.

In some embodiments, the method comprises coupling a second electrode to the substrate that can charge the substrate to a second electric potential.

In some embodiments, the method comprises providing a chamber enclosing the discharged spray wherein the chamber comprises an insulator material.

In some embodiments, the coated substrates are produced at a rate of 10 or more substrates every hour.

A device comprising a substrate; a plurality of layers deposited on said stent framework to form said coronary stent; wherein at least one of said layers comprises a polymer comprising poly(D,L-lactic-co-glycolic acid) substantially free of acidic impurities and at least one of said layers comprises rapamycin; wherein at least part of rapamycin is in crystalline form and said rapamycin is provided at a reduced dose compared to a conventional drug eluting stent.

In some embodiments, the rapamycin and polymer are in the same layer; in separate layers or form overlapping layers.

In some embodiments, the plurality of layers comprise five layers deposited as follows: a first polymer layer, a first rapamycin layer, a second polymer layer, a second rapamycin layer and a third polymer layer.

In some embodiments, the substrate is a biomedical implant selected from the group consisting of stents, joints, screws, rods, pins, plates, staples, shunts, clamps, clips, sutures, suture anchors, electrodes, catheters, leads, grafts, dressings, pacemakers, pacemaker housings, cardioverters, cardioverter housings, defibrillators, defibrillator housings, prostheses, ear drainage tubes, ophthalmic implants, orthopedic devices, vertebral disks, bone substitutes, anastomotic devices, perivascular wraps, colostomy bag attachment devices, hemostatic barriers, vascular implants, vascular supports, tissue adhesives, tissue sealants, tissue scaffolds and intraluminal devices.

A device, comprising: a stent framework; and a rapamycin-polymer coating wherein at least part of rapamycin is in crystalline form and the rapamycin-polymer coating comprises one or more resorbable polymers and said rapamycin is provided at a reduced dose compared to a conventional drug eluting stent, and wherein the resorbable polymer comprises poly(D,L-lactic-co-glycolic acid) substantially free of acidic impurities.

In some embodiments, the rapamycin-polymer coating has substantially uniform thickness and rapamycin in the coating is substantially uniformly dispersed within the rapamycin-polymer coating.

In some embodiments, at least part of said rapamycin forms a phase separate from one or more phases formed by said polymer.

In some embodiments, the rapamycin is at least 50% crystalline. In some embodiments, the rapamycin is at least 75% crystalline. In some embodiments, the rapamycin is at least 90% crystalline. In some embodiments, the rapamycin is at least 95% crystalline.In some embodiments, the rapamycin is at least 99% crystalline.

In some embodiments, the polymer is a mixture of two or more polymers. In some embodiments, the mixture of polymers forms a continuous film around particles of rapamycin. In some embodiments, the two or more polymers are intimately mixed. In some embodiments, the mixture comprises no single polymer domain larger than about 20 nm. In some embodiments, each polymer in said mixture comprises a discrete phase.

In some embodiments, the discrete phases formed by said polymers in said mixture are larger than about 10nm. In some embodiments, the discrete phases formed by said polymers in said mixture are larger than about 50nm.

In some embodiments, the rapamycin in said stent has a shelf stability of at least 3 months.

In some embodiments, the rapamycin in said stent has a shelf stability of at least 6 months.

In some embodiments, the rapamycin in said stent has a shelf stability of at least 12 months.

In some embodiments, the coating is substantially conformal.

In some embodiments, the device provides an elution profile wherein about 10% to about 50% of rapamycin is eluted at week 1 after the composite is implanted in a subject under physiological conditions, about 25% to about 75% of rapamycin is eluted at week 2 and about 50% to about 100% of rapamycin is eluted at week 6.

In some embodiments, the device provides an elution profile wherein about 10% to about 50% of rapamycin is eluted at week 1 after the composite is implanted in a subject under physiological conditions, about 20% to about 75% of rapamycin is eluted at week 2 and about 50% to about 100% of rapamycin is eluted at week 10.

In some embodiments, the device provides an elution profile comparable to first order kinetics.

In some embodiments, the device provides elution profile control

In some embodiments, the device provides tissue concentration control.

In some embodiments, the device provides tissue concentration of at least twice the tissue concentration provided by a conventional stent.

In some embodiments, the device provides tissue concentration of at least 5 times greater than the tissue concentration provided by a conventional stent.

In some embodiments, the device provides tissue concentration of at least 10 times greater than the tissue concentration provided by a conventional stent.

In some embodiments, the device provides tissue concentration of at least 15 times greater than the tissue concentration provided by a conventional stent.

In some embodiments, the device provides tissue concentration of at least 20 times greater than the tissue concentration provided by a conventional stent.

In some embodiments, the device provides tissue concentration of at least 50

In some embodiments, the device provides tissue concentration of at least 100 times greater than the tissue concentration provided by a conventional stent.

In some embodiments, the polymer is resorbed within 45-90 days after an angioplasty procedure.

In some embodiments, the device provides reduced inflammation over the course of polymer resorbtion compared to a conventional stent.

Provided herein is a method of preparing a coated device comprising: providing a substrate; depositing a plurality of layers on said substrate to form said coated device; wherein at least one of said layers comprises a drug-polymer coating wherein at least part of the drug is in crystalline form and the polymer is a bioabsorbable polymer comprising poly(D,L-lactic-co-glycolic acid) substantially free of acidic impurities.

In some embodiments, the substrate is a stent framework.

In some embodiments, the drug and polymer are in the same layer; in separate layers or in overlapping layers.

In some embodiments, the substrate is made of stainless steel. In some embodiments, the substrate is formed from a metal alloy. In some embodiments, the substrate is formed from a cobalt chromium alloy. In some embodiments, the substrate has a thickness of about 50% or less of a thickness of the coronary stent.

In some embodiments, the substrate has a thickness of about 100 µm or less.

In some embodiments, the method comprises depositing 4 or more layers. In some embodiments, the method comprises depositing 10, 20, 50, or 100 layers. In some embodiments, the method comprises depositing at least one of: at least 10, at least 20, at least 50, and at least 100 layers.

In some embodiments, the drug comprise a macrolide immunosuppressive (limus) drug. In some embodiments, the macrolide immunosuppressive drug comprises one or more of rapamycin, biolimus (biolimus A9), 40-O-(2-Hydroxyethyl)rapamycin (everolimus), 40-O-Benzyl-rapamycin, 40-O-(4'-Hydroxymethyl)benzyl-rapamycin, 40-O-[4'-(1,2-Dihydroxyethyl)]benzyl-rapamycin, 40-O-Allyl-rapamycin, 40-O-[3'-(2,2-Dimethyl-1,3-dioxolan-4(S)-yl)-prop-2'-en-1'-yl]-rapamycin, (2':E,4'S)-40-O-(4',5'-Dihydroxypent-2'-en-1'-yl)-rapamycin 40-O-(2-Hydroxy)ethoxycar-bonylmethyl-rapamycin, 40-O-(3-Hydroxy)propyl-rapamycin 40-O-(6-Hydroxy)hexyl-rapamycin 40-O-[2-(2-Hydroxy)ethoxy]ethyl-rapamycin 4O-O-[(3S)-2,2-Dimethyldioxolan-3-yl]methyl-rapamycin, 40-O-[(2S)-2,3-Dihydroxyprop-1-yl]-rapamycin, 4O-O-(2-Acetoxy)ethyl-rapamycin 4O-O-(2-Nicotinoyloxy)ethyl-rapamycin, 4O-O-[2-(N-Morpholino)acetoxy]ethyl-rapamycin 4O-O-(2-N-Imidazolylacetoxy)ethyl-rapamycin, 40-O- [2-(N-Methyl-N'-piperazinyl)acetoxy]ethyl-rapamycin, 39-O-Desmethyl-39,40-O,O-ethylene-rapamycin, (26R)-26-Dihydro-40-O-(2-hydroxy)ethyl-rapamycin, 28-O-Methyl-rapamycin, 4O-O-(2-Aminoethyl)-rapamycin, 4O-O-(2-Acetaminoethyl)-rapamycin 4O-O-(2-Nicotinamidoethyl)-rapamycin, 4O-O-(2-(N-Methyl-imidazo-2'-ylcarbethoxamido)ethyl)-rapamycin, 4O-O-(2-Ethoxycarbonylaminoethyl)-rapamycin, 40-O-(2-Tolylsulfonamidoethyl)-rapamycin, 40-O-[2-(4',5'-Dicarboethoxy-1',2',3'-triazol-1'-yl)-ethyl]-rapamycin, 42-Epi-(tetrazolyl)rapamycin (tacrolimus), and 42-[3-hydroxy-2-(hydroxymethyl)-2-methylpropanoate]rapamycin (temsirolimus), (42S)-42-Deoxy-42-(1H-tetrazol-1-yl)-rapamycin (zotarolimus), and salts, derivatives, isomers, racemates, diastereoisomers, prodrugs, hydrate, ester, or analogs thereof.

In some embodiments, the macrolide immunosuppressive drug is at least 50% crystalline.

In some embodiments, depositing a plurality of layers on said stent framework to form said coronary stent comprises depositing polymer particles on said framework by an RESS process. In some embodiments, depositing a plurality of layers on said stent framework to form said coronary stent comprises depositing polymer particles on said framework in dry powder form.

Provided herein is a coated stent, comprising: a stent framework; a first layer of bioabsorbable polymer; and a rapamycin-polymer coating comprising rapamycin and a second bioabsorbable polymer wherein at least part of rapamycin is in crystalline form and wherein the first polymer is a slow absorbing polymer and the second polymer is a fast absorbing polymer, and wherein at least one of the first polymer and the second polymer is poly(D,L-lactic-co-glycolic acid) substantially free of acidic impurities.

### EXAMPLES

The following examples are given to enable those skilled in the art to more clearly understand and to practice the present invention. They should not be considered as limiting the scope of the invention, but merely as being illustrative and representative thereof. The following examples are provided to illustrate selected embodiments. They should not be considered as limiting the scope of the invention, but merely as being illustrative and representative thereof. Thus, the examples provided below, while illustrated with a particular medical device or active agent, are applicable to the range of medical devices and active agents described herein.

### EXAMPLE 1- PREPARATION OF AN ORGANIC SOLUTION OF POLY(D,L-LACTIC-CO-GLYCOLIC ACID) FREE OF ACIDIC IMPURTIES.

A dry reaction chamber under dry argon is charged with 2 parts CaH₂, 10 parts poly(D,L-lactic-co-glycolic acid) and 88 parts anhydrous tetrahydrofuran. After stirring at room temperture overnight, the suspension is filtered with the exclusion of moisture and the solution of poly(D,L-lactic-co-glycolic acid) in tetrahydrofuran is used directly in the next process. Alternatively, the tetrahydrofuran is removed under reduced pressure and resisdual poly(D,L-lactic-co-glycolic acid) is used.

### EXAMPLE 2 - PREPARATION OF AN FLUOROCARBON SOLUTION OF POLY(D,L-LACTIC-CO-GLYCOLIC ACID) FREE OF ACIDIC IMPURTIES.

Into a dry reaction chamber equipped for magnetic stirring and suitable for maintenance of a supercritical fluid state is placed a cylindrical screen of a fine 304 stainless steel woven wire cloth/screen (635X635 mesh; 0.0009" wire diameter; with a percentage of open area range of 15% ± 5%) that has entrapped behind it a slight excess of CaH₂. This chamber is then charged with 10 parts poly(D,L-lactic-co-glycolic acid) and 88 parts FC236. The chamber is pressurized sufficient to create a supercritical state, stirring is begun and after 24 hours, the FC236 solution is dispensed from the reaction chamber and used directly in the next process.

### EXAMPLE 3 - PREPARATION OF POLY(D,L-LACTIC-CO-GLYCOLIC ACID) FREE OF ACIDIC IMPURTIES.

A heated column is charged with CaH₂ and flushed with nitrogen gas. Poly(D,L-lactic-co-glycolic acid) is warmed to provide a liquid solution and is passed through the calicum hydride column. Upon eluting from the column, the poly(D,L-lactic-co-glycolic acid) is protected from mosisture and used directly in the next process.

### EXAMPLE 4 - PREPARATION OF POLY(D,L-LACTIC-CO-GLYCOLIC ACID) FREE OF ACIDIC IMPURTIES USING ELECTROPHORESIS.

The electrophoresis gel is saturated with a simple phosphate buffer and supported by a nylon mesh. The polymer is dissolved in a solution of dimethyl formamide and poured on one side of the chamber, the phosphate buffer on the other. Electrodes are positioned on each side of the chamber and an electric current is passed through the partitioned gels. The electrodes are arranged in such a way that the anions to flow toward the anode.

The potential is adjusted and after 2 hours the acrylamide contains more that 95% of the original catalytic quantities of acid moieties. It is discarded. The polymer is recovered by precipitation using a copious amount of methanol. The polymer is dried to constant weight under a stream of dry nitrogen.

### EXAMPLE 5 - DETERMINATION OF TISSUE CONCENTRATION.

In-vivo testing: A group of 27 New Zealand white rabbits is prepared for a Seldinger procedure using a cutting balloon coated with a formulation as described herein and sirolimus with total loading of sirolimus ∼20 µg with the coating preferentially on the wire of the cutting balloon. The device is placed at a coronary artery intervention site with the assistance of fluoroscopy to aid in positioning the device at the same location in each subject. Six animals are subjected to the procedure using a coated balloon that does not have sirolimus in the coating. After deployment and removal of the device, 3 control animals are sacrificed at 1 hour post deployment and serum and tissue samples are collected. The 3 remaining control animals are sacrificed at 56 days post deployment. During the course of the study, serum samples are collected from control and drug-treated animals every five days. The drug treated animals, 3 each, are sacrificed at 1 hour, 24 hours, 7 days, 14 days, 28 days, 42 days and 56 days post deployment. A serum sample as well as a tissue sample from the deployment site is collected.

The tissue and serum samples may be subjected to analysis for sirolimus concentration. In order to determine the amount of coating freed from the device and/or delivered to the intervention site as a percent of the total amount of coating on the substrate, the tissue concentration of sirolimus at the one hour time point (or any time point within the first day following of the procedure) may be used used along with the total content expected for the coating (based on the total content for the manufacturing lot) or along with the content of coating remaining on the device once removed and the percentage calculated. This percentage is correlative of the percent of coating freed, dissociated, and/or transferred from the device and delivered to the intervention site. Alternatively, the tissue may be analyzed by various means (noted herein, including but not limited to SEM, TEM, and, where image enhanced polymers are used, various imaging means capable of detecting these enhanced polymers) to detect the percent of the coating freed, dissociated and/or transferred from the substrate and delivered to the intervention site. Again, the amount of coating known to be on the substrate based on manufacturing lot characteristics, and/or an assessment of the coating remaining on the device following removal of the device from the subject (for example, wherein the device is an angioplasty catheter and the substrate is the balloon of the catheter) may be used to determine the percent of coating freed, dissociated, and/or transferred from the device. In some instances, an assessment of the device following the procedure alone is sufficient to assess the amount freed or dissociated from the substrate, without determination of the amount delivered to the intervention site. Additionally, where a determination of improvement and/or disease treatment is desired, levels of proinflammatory markers could be tested to show improvement and/or treatment of a disease and/or ailment, for example, by testing high sensitive C-reactive protein (hsCRP), interleukin-6 (IL-6), interleukin-1β (IL-1β), and/or monocyte chemoattractant protein-1 (MCP-1). The release kinetics of the drug may be shown by plotting the sirolimus concentrations at the timepoints noted above.

### EXAMPLE 6 - DETERMINATION OF IN VITRO RELEASE PROFILE.

In-vitro testing: One sample of the coated compliant balloon prepared by the methods described herein is secured to a balloon catheter. A segment of optically clear TYGON® B-44-3 tubing with O.D. = 0.125", I.D. = 0.0625" (Available from McMaster-Carr Part Number: 5114K11 (www.mcmaster.com)) is filled with phosphate-buffered saline solution and immersed in a water bath at 37 °C to mimic physiological conditions of deployment into a subject. The coated balloon is inserted into the tubing and the balloon is inflated to at least 25% below the balloon's nominal pressure to mechanically transfer the coating from the balloon to the tubing wall. The balloon is deflated and removed from the tubing. Optical microscopy is performed on the tubing and/or the balloon (which is inflated to at least 25% below the balloon's nominal pressure, at least) to determine the presence and amount of coating transferred to the tubing and/or the amount of coating freed, dissociated, and/or transferred from the balloon.

Method for the determination of sirolimus levels: Media may be assayed for sirolimus content using HPLC. Calibration standards containing known amounts of drug are to determine the amount of drug eluted. The multiple peaks present for the sirolimus (also present in the calibration standards) are added to give the amount of drug eluted at that time period (in absolute amount and as a cumulative amount eluted). HPLC analysis is performed using Waters HPLC system, set up and run on each sample as provided in the Table 1 below using an injection volume of 100 L.

**Table 1**

| Time point (minutes) | % Acetonitrile | % Ammonium Acetate (0.5%), pH 7.4 | Flow Rate (mL/min) |
|---|---|---|---|
| 0.00 | 10 | 90 | 1.2 |
| 1.00 | 10 | 90 | 1.2 |
| 12.5 | 95 | 5 | 1.2 |
| 13.5 | 100 | 0 | 1.2 |
| 14.0 | 100 | 0 | 3 |
| 16.0 | 100 | 0 | 3 |
| 17.0 | 10 | 90 | 2 |
| 20.0 | 10 | 90 | 0 |

In-vitro Coating test: One sample of the coated compliant balloon prepared as described herein is secured to a balloon catheter. A segment of optically clear TYGON® B-44-3 tubing with O.D. = 0.125", I.D. = 0.0625" (Available from McMaster-Carr Part Number: 5114K11 (www.mcmaster.com)) is filled with phosphate-buffered saline solution and immersed in a water bath at 37 °C to mimic physiological conditions of deployment into a subject. The coated balloon is inserted into the tubing and the balloon is inflated to at least 25% below the balloon's nominal pressure to mechanically transfer the coating from the balloon to the tubing wall. The balloon is deflated and removed from the tubing. The section of tubing exposed to the deployed balloon is cut away from the remainder of the tubing and the interior of the excised tubing rinsed with a small amount of ethanol and an amount of methylene chloride to make up 25 mL total volume of rinsings which are collected in a flask for analysis. Analysis by HPLC as described above is performed to determine the amount of material freed, dissociated, and/or transferred from the balloon. This analysis may instead and/or alternatively include testing of the substrate itself to determine the amount of coating freed, dissociated, and/or transferred from the device during this in-vitro test.

In-vitro testing of release kinetics: One sample of the coated compliant balloon with total loading of sirolimus ∼20 µg prepared by the methods described herein is secured to a balloon catheter. A flask containing exactly 25 mL of pH 7.4 aqueous phosphate buffer equilibrated to 37 °C equipped for magnetic stirring is prepared. Into this flask is placed the coated balloon and the catheter portion of the apparatus is secured such that the balloon does not touch the sides of the flask. The balloon is inflated to 120 psi with sterile water. Aliquots of 100 L are removed prior to addition of the balloon, after placement of the balloon but prior to inflation of the balloon, and at regular time intervals of 2, 4, 6, 8, 10, 12, and 14 minutes. Upon removal of each aliquot an equivalent volume of aqueous buffer is added to maintain the volume at 25 mL. The aliquots are analyzed by HPLC as described above for the concentration of sirolimus.

### EXAMPLE 7: CRYSTALLINITY OF DRUG ON A DEVICE

The presence and or quantification of the active agent crystallinity can be determined from a number of characterization methods known in the art, but not limited to, XRPD, vibrational spectroscopy (FTIR, NIR, Raman), polarized optical microscopy, calorimetry, thermal analysis and solid-state NMR.

### X-Ray Diffraction to Determine the Presence and/or Quantification of Active Agent Crystallinity

Active agent and polymer coated proxy substrates are prepared using 316L stainless steel coupons for X-ray powder diffraction (XRPD) measurements to determine the presence of crystallinity of the active agent. The coating on the coupons is equivalent to the coating on the stents described herein. Coupons of other materials described herein, such as cobalt-chromium alloys, may be similarly prepared and tested. Likewise, substrates such as stents, or other medical devices described herein may be prepared and tested. Where a coated stent is tested, the stent may be cut lengthwise and opened to lay flat in a sample holder.

For example XRPD analyses are performed using an X-ray powder diffractometer (for example, a Bruker D8 Advance X-ray diffractometer) using Cu Kα radiation. Diffractograms are typically collected between 2 and 40 degrees 2 theta. Where required low background XRPD sample holders are employed to minimize background noise.

The diffractograms of the deposited active agent are compared with diffractograms of known crystallized active agents, for example micronized crystalline sirolimus in powder form. XRPD patterns of crystalline forms show strong diffraction peaks whereas amorphous show diffuse and non-distinct patterns. Crystallinity is shown in arbitrary Intensity units.

A related analytical technique which may also be used to provide crystallinity detection is wide angle scattering of radiation (e.g.; Wide Anle X-ray Scattering or WAXS), for example, as described in F. Unger, et al., "Poly(ethylene carbonate): A thermoelastic and biodegradable biomaterial for drug eluting stent coatings?" Journal of Controlled Release, Volume 117, Issue 3, 312-321 (2007) for which the technique and variations of the technique specific to a particular sample would be obvious to one of skill in the art.

### Raman Spectroscopy

Raman spectroscopy, a vibrational spectroscopy technique, can be useful, for example, in chemical identification, characterization of molecular structures, effects of bonding, identification of solid state form, environment and stress on a sample. Raman spectra can be collected from a very small volume (< 1 µm³ ); these spectra allow the identification of species present in that volume. Spatially resolved chemical information, by mapping or imaging, terms often used interchangeably, can be achieved by Raman microscopy.

Raman spectroscopy and other analytical techniques such as described in Balss, et al., "Quantitative spatial distribution of sirolimus and polymers in drug-eluting stents using confocal Raman microscopy" J. of Biomedical Materials Research Part A, 258-270 (2007), incorporated in its entirety herein by reference, and/or described in Belu et al., "Three-Dimensional Compositional Analysis of Drug Eluting Stent Coatings Using Cluster Secondary Ion Mass Spectroscopy" Anal. Chem. 80: 624-632 (2008) incorporated herein in its entirety by reference may be used.

For example, to test a sample using Raman microscopy and in particular confocal Raman microscopy, it is understood that to get appropriate Raman high resolution spectra sufficient acquisition time, laser power, laser wavelength, sample step size and microscope objective need to be optimized. For example a sample (a coated stent) is prepared as described herein. Alternatively, a coated coupon could be tested in this method. Maps are taken on the coating using Raman microscopy. A WITec CRM 200 scanning confocal Raman microscope using a Nd:YAG laser at 532 nm is applied in the Raman imaging mode. The laser light is focused upon the sample using a 100× dry objective (numerical aperture 0.90), and the finely focused laser spot is scanned into the sample. As the laser scans the sample, over each 0.33 micron interval a Raman spectrum with high signal to noise is collected using 0.3 seconds of integration time. Each confocal cross-sectional image of the coatings displays a region 70 µm wide by 10 µm deep, and results from the gathering of 6300 spectra with a total imaging time of 32 min.

Multivariate analysis using reference spectra from samples of rapamycin (amorphous and crystalline) and polymer are used to deconvolve the spectral data sets, to provide chemical maps of the distribution.

### Infrared (IR) Spectroscopy for In-Vitro Testing

Infrared (IR) Spectroscopy such as FTIR and ATR-IR are well utilized techniques that can be applied to show, for example, the quantitative drug content, the distribution of the drug in the sample coating, the quantitative polymer content in the coating, and the distribution of polymer in the coating. Infrared (IR) Spectroscopy such as FTIR and ATR-IR can similarly be used to show, for example, drug crystallinity. The following table (Table 2) lists the typical IR materials for various applications. These IR materials are used for IR windows, diluents or ATR crystals.

**Table 2**

| MATERIAL | NACL | KBR | CSI | AGCL | GE | ZNSE | DIAMOND |
|---|---|---|---|---|---|---|---|
| Transmission range (cm-1) | 40,000 ∼625 | 40,000 ∼400 | 40,000 ∼200 | 25,000 ∼360 | 5,500 ∼625 | 20,000 ∼454 | 40,000 ∼2,500 & 1667-33 |
| Water sol (g/100g, 25C) | 35.7 | 53.5 | 44.4 | Insol. | Insol. | Insol. | Insol. |
| Attacking materials | Wet Solvents | Wet Solvents | Wet Solvents | Ammonium Salts | H2SO4, aqua regin | Acids, strong alkalies, chlorinated solvents | K2Cr2Os, conc. H2SO4 |

In one test, a coupon of crystalline ZnSe is coated by the processes described herein, creating a PDPDP (Polymer, Drug, Polymer, Drug, Polymer) layered coating that is about 10 microns thick. The coated coupon is analyzed using FTIR. The resulting spectrum shows crystalline drug as determined by comparison to the spectrum obtained for the crystalline form of a drug standard (i.e. a reference spectrum).

### Differential Scanning Calorimetry (DSC)

DSC can provide qualitative evidence of the crystallinity of the drug (e.g. rapamycin) using standard DSC techniques obvious to one of skilled in the art. Crystalline melt can be shown using this analytical method (e.g. rapamycin crystalline melting - at about 185 °C to 200 °C, and having a heat of fusion at or about 46.8 J/g). The heat of fusion decreases with the percent crystallinity. Thus, the degree of crystallinity could be determined relative to a pure sample, or versus a calibration curve created from a sample of amorphous drug spiked and tested by DSC with known amounts of crystalline drug. Presence (at least) of crystalline drug on a stent could be measured by removing (scraping or stripping) some drug from the stent and testing the coating using the DSC equipment for determining the melting temperature and the heat of fusion of the sample as compared to a known standard and/or standard curve.

### Confocal Raman Microscopy

Confocal Raman Microscopy can provide nondestructive depth analysis and allows coating specific Raman spectral features to be obtained (Bugay et al., "Raman Analysis of Pharmaceuticals," in "Applications of Vibrational Spectroscopy in Pharmaceutical Research and Development," Ed. Pivonka, D. E., Chalmers, J. M., Griffiths, P. R. (2007) Wiley and Sons*).* In confocal Raman microscopy an aperture is place in a focal place of the collected beam. This limitation defines a shallow portion of the depth of field and thereby provides definition of the z-axis spatial resolution for data collection. By adjusting the aperture and moving the focus within the sample, the sampling position within the sample moves. Moving the sample focus from the top surface, deeper into the specimen facilitates nondestructive depth analysis.

### EXAMPLE 8: DETECTION OF COATING REMAINING ON A DEVICE FOLLOWING USE

The ability to uniformly coat a device with controlled composition and thickness using electrostatic capture in a rapid expansion of supercritical solution (RESS) experimental series has been demonstrated.

The coating remaining on a device following use of the device may be examined by any of the following test methods. For example, the coating remaining on a device following use is an indication of the maximum amount of coating freed from the device. In an in-vivo or in-vitro method, an embodiment of the device that is removed from the subject once used is tested for remaining coating (for example, a balloon).

### Scanning Electron Microscopy (SEM)

Stents are observed by SEM using a Hitachi S-4800 with an accelerating voltage of 800V. Various magnifications are used to evaluate the integrity, especially at high strain regions. SEM can provide top-down and cross-section images at various magnifications. Coating uniformity and thickness can also be assessed using this analytical technique.

Pre- and post-expansions stents are observed by SEM using a Hitachi S-4800 with an accelerating voltage of 800V. Various magnifications are used to evaluate the integrity of the layers, especially at high strain regions.

### Scanning Electron Microscopy (SEM) with Focused Ion Beam (FIB)

Stents as described herein, and or produced by methods described herein are visualized using SEM-FIB analysis. Alternatively, a coated coupon could be tested in this method. Focused ion beam FIB is a tool that allows precise site-specific sectioning, milling and depositing of materials. FIB can be used in conjunction with SEM, at ambient or cryo conditions, to produce in-situ sectioning followed by high-resolution imaging. Cross-sectional FIB images may be acquired, for example, at 7000x and/or at 20000x magnification. An even coating of consistent thickness is visible.

### Optical Microscopy

An Optical micrscope may be used to create and inspect the stents and to empirically survey the coating of the substrate (e.g. coating uniformity). Nanoparticles of the drug and/or the polymer can be seen on the surfaces of the substrate using this analytical method. Following sintering, the coatings can be see using this method to view the coating conformaliy and for evidence of crystallinity of the drug.

In-vitro test: One sample of the coated compliant balloon prepared in Example 1 is secured to a balloon catheter. A segment of optically clear TYGON® B-44-3 tubing with O.D. = 0.125", I.D. = 0.0625" (Available from McMaster-Carr Part Number: 5114K11 (www.mcmaster.com)) is filled with phosphate-buffered saline solution and immersed in a water bath at 37 °C to mimic physiological conditions of deployment into a subject. The coated balloon is inserted into the tubing and the balloon is inflated to at least 25% below the balloon's nominal pressure to mechanically transfer the coating from the balloon to the tubing wall. The balloon is deflated and removed from the tubing. Scanning Electron Microscopy is performed on the tubing and the balloon (which is inflated to at least 25% below the balloon's nominal pressure, at least) to determine the presence and amount of coating transferred to the tubing and/or the amount of coating freed, dissociated, and/or transferred from the balloon.

### EXAMPLE 9: DETERMINATION AND DETECTION OF COATING CONFORMALITY

The ability to uniformly coat devices, e.g., pre- and post-expansion stents, and balloons, with controlled composition and thickness using electrostatic capture in a rapid expansion of supercritical solution (RESS) experimental series has been demonstrated.

### Scanning Electron Microscopy (SEM)

Devices are observed by SEM using a Hitachi S-4800 with an accelerating voltage of 800V. Various magnifications are used to evaluate the integrity, especially at high strain regions. SEM can provide top-down and cross-section images at various magnifications. Coating uniformity and thickness can also be assessed using this analytical technique.

Pre- and post-inflation balloons, for example, may be observed by SEM using a Hitachi S-4800 with an accelerating voltage of 800V. Various magnifications may be used to evaluate the integrity of the layers, and or of the coating.

### Scanning Electron Microscopy (SEM) with Focused Ion Beam (FIB)

Devices as described herein, and or produced by methods described herein are visualized using SEM-FIB analysis. Alternatively, a coated coupon could be tested in this method. Focused ion beam FIB is a tool that allows precise site-specific sectioning, milling and depositing of materials. FIB can be used in conjunction with SEM, at ambient or cryo conditions, to produce in-situ sectioning followed by high-resolution imaging. Cross-sectional FIB images may be acquired, for example, at 7000x and/or at 20000x magnification. An even coating of consistent thickness is visible.

### Optical Microscopy

An optical microscope may be used to create and inspect the devices and to empirically survey the coating of the substrate (e.g. coating uniformity). Nanoparticles of the drug and/or the polymer can be seen on the surfaces of the substrate using this analytical method. Following sintering, the coatings can be see using this method to view the coating conformality and for evidence of crystallinity of the drug.

### EXAMPLE 10: VISUALIZATION OF POLYMER/ACTIVE AGENT LAYERS COATING A DEVICE

### Raman Spectroscopy

As discussed herein, Raman spectroscopy can be applied to characterize the chemical structure and relative concentrations of drug and polymer coatings. For example, confocal Raman Spectroscopy / microscopy can be used to characterize the relative drug to polymer ratio at the outer ~ 1µm of the coated surface. In addition confocal Raman x-z or z (maps or line scans) microscopy can be applied to characterize the relative drug to polymer ratio as a function of depth. Additionally cross-sectioned samples can be analysed. Raman spectroscopy and other analytical techniques such as described in Balss, et al., "Quantitative spatial distribution of sirolimus and polymers in drug-eluting stents using confocal Raman microscopy" J. of Biomedical Materials Research Part A, 258-270 (2007), incorporated in its entirety herein by reference, and/or described in Belu et al., "Three-Dimensional Compositional Analysis of Drug Eluting Stent Coatings Using Cluster Secondary Ion Mass Spectroscopy" Anal. Chem. 80: 624-632 (2008) incorporated herein in its entirety by reference may be used.

A sample (a coated substrate) is prepared as described herein. Images are taken on the coating using Raman Spectroscopy. Alternatively, a coated coupon could be tested in this method. To test a sample using Raman microscopy and in particular confocal Raman microscopy, it is understood that to get appropriate Raman high resolution spectra sufficient acquisition time, laser power, laser wavelength, sample step size and microscope objective need to be optimized.

For example a WITec CRM 200 scanning confocal Raman microscope using a Nd:YAG laser at 532 nm is applied in the Raman imaging mode to give x-z maps. The sample is placed upon a piezoelectrically driven table, the laser light is focused upon the sample using a 100× dry objective (numerical aperture 0.90), and the finely focused laser spot is scanned into the sample. As the laser scans the sample, over each 0.33 micron interval a Raman spectrum with high signal to noise is collected using 0.3 Seconds of integration time. Each confocal cross-sectional image of the coatings displays a region 70 µm wide by 10 µm deep, and results from the gathering of 6300 spectra with a total imaging time of 32 min. Multivariate analysis using reference spectra from samples of rapamycin and polymer are used to deconvolve the spectral data sets, to provide chemical maps of the distribution.

In another test, spectral depth profiles (x-z maps) of samples are performed with a CRM200 microscope system from WITec Instruments Corporation (Savoy, IL). The instrument is equipped with a Nd:YAG frequency doubled laser (532 excitation), a single monochromator (Acton) employing a 600 groove/mm grating and a thermoelectrically cooled 1024 by 128 pixel array CCD camera (Andor Technology). The microscope is equipped with appropriate collection optics that include a holographic laser bandpass rejection filter (Kaiser Optical Systems Inc.) to minimize Rayleigh scatter into the monochromator. The Raman scattered light are collected with a 50 micron optical fiber. Using the "Raman Spectral Imaging" mode of the instrument, spectral images are obtained by scanning the sample in the x, z direction with a piezo driven xyz scan stage and collecting a spectrum at every pixel. Typical integration times are 0.3s per pixel. The spectral images are 4800 total spectra corresponding to a physical scan dimension of 40 by 20 microns. For presentation of the confocal Raman data, images are generated based on unique properties of the spectra (i.e. integration of a Raman band, band height intensity, or band width). The microscope stage is modified with a custom-built sample holder that positioned and rotated the stents around their primary axis. The x direction is defined as the direction running parallel to the length of the stent and the z direction refers to the direction penetrating through the coating from the air-coating to the coating-metal interface. Typical laser power is <10mW on the sample stage. All experiments can be conducted with a plan achromat objective, 100 x NA = 0.9 (Nikon).

Samples (n=5) comprising metal substrates made of L605 (0.05-0.15% C, 1.00-2.00% Mn, maximum 0.040% Si, maximum 0.030% P, maximum 0.3% S, 19.00-21.00% Cr, 9.00-11.00% Ni, 14.00-16.00% W, 3.00% Fe, and Bal. Co) and having coatings as described herein and/or produced by methods described herein can be analyzed. For each sample, three locations are selected along the substrate length. The three locations are located within one-third portions of the substrates so that the entire length of the substrate are represented in the data. The stent is then rotated 180 degrees around the circumference and an additional three locations are sampled along the length. In each case, the data is collected from the strut portion of the substrate. Six random spatial locations are also profiled on coated coupon samples made of L605 and having coatings as described herein and/or produced by methods described herein. The Raman spectra of each individual component present in the coatings are also collected for comparison and reference. Using the instrument software, the average spectra from the spectral image data are calculated by selecting the spectral image pixels that are exclusive to each layer. The average spectra are then exported into GRAMS/AI v. 7.02 software (Thermo Galactic) and the appropriate Raman bands are fit to a Voigt function. The band areas and shift positions are recorded.

The pure component spectrum for each component of the coating (e.g. drug, polymer) are also collected at 532 and 785 nm excitation. The 785 nm excitation spectra are collected with a confocal Raman microscope (WITec Instruments Corp. Savoy, IL) equipped with a 785 nm diode laser, appropriate collection optics, and a back-illuminated thermoelectriaclly cooled 1024 x 128 pixel array CCD camera optimized for visible and infrared wavelengths (Andor Technology).

### X-ray photoelectron spectroscopy (XPS)

XPS can be used to quantitatively determine elemental species and chemical bonding environments at the outer 5-10nm of sample surface. The technique can be operated in spectroscopy or imaging mode. When combined with a sputtering source XPS can be utilized to give depth profiling chemical characterization. XPS (ESCA) and other analytical techniques such as described in Belu et al., "Three-Dimensional Compositional Analysis of Drug Eluting Stent Coatings Using Cluster Secondary Ion Mass Spectroscopy" Anal. Chem. 80: 624-632 (2008) incorporated herein in its entirety by reference may be used.

For example, in one test, a sample comprising a stent coated by methods described herein and/or a device as described herein is obtained. XPS analysis is performed on a sample using a Physical Electronics Quantum 2000 Scanning ESCA. The monochromatic A1 Kα source is operated at 15 kV with a power of 4.5 W. The analysis is done at a 45° take off angle. Three measurements are taken along the length of each sample with the analysis area ∼ 20 microns in diameter. Low energy electron and Ar⁺ ion floods are used for charge compensation.

### Time of Flight Secondary Ion Mass Spectrometery (TOF-SIMS)

TOF-SIMS can be used to determine molecular species (drug and polymer) at the outer 1-2nm of sample surface when operated under static conditions. The technique can be operated in spectroscopy or imaging mode at high spatial resolution. Additionally cross-sectioned samples can be analysed. When operated under dynamic experimental conditions, known in the art, depth profiling chemical characterization can be achieved.

For example, to analyze the uppermost surface only, static conditions (for example a ToF-SIMS IV (IonToF, Munster)) using a 25Kv Bi++ primary ion source maintained below 1012 ions per cm2 is used.. Where necessary a low energy electron flood gun (0.6 nA DC) is used to charge compensate insulating samples.

Cluster Secondary Ion Mass Spectrometry, may be employed for depth profiling as described Belu et al., "Three-Dimensional Compositional Analysis of Drug Eluting Stent Coatings Using Cluster Secondary Ion Mass Spectroscopy" Anal. Chem. 80: 624-632 (2008) incorporated herein in its entirety by reference.

For example, a ballon coated as described herein is obtained. The balloon is prepared for SIMS analysis by cutting it longitudinally and opening it up with tweezers. The balloon is then pressed into multiple layers of indium foil with the outer diameter facing outward.

TOF-SIMS depth profiling experiments are performed using an Ion-TOF IV instrument equipped with both Bi and SF5+ primary ion beam cluster sources. Sputter depth profiling is performed in the dual-beam mode, whilst preserving the chemical integrity of the sample. The analysis source is a pulsed, 25-keV bismuth cluster ion source, which bombarded the surface at an incident angle of 45° to the surface normal. The target current is maintained at ∼0.3 pÅ (+10%) pulsed current with a raster size of 200 um x 200 um for all experiments. Both positive and negative secondary ions are extracted from the sample into a reflectron-type time-of-flight mass spectrometer. The secondary ions are then detected by a microchannel plate detector with a post-acceleration energy of 10 kV. A low-energy electron flood gun is utilized for charge neutralization in the analysis mode.

The sputter source used is a 5-keV SF5+ cluster source also operated at an incident angle of 45° to the surface normal. For thin model samples on Si, the SF5+ current is maintained at ∼2.7 nÅ with a 750 um x 750 um raster. For the thick samples on coupons and for the samples on stents, the current is maintained at 6nA with a 500 um x 500 um raster. All primary beam currents are measured with a Faraday cup both prior to and after depth profiling.

All depth profiles are acquired in the noninterlaced mode with a 5-ms pause between sputtering and analysis. Each spectrum is averaged over a 7.37 second time period. The analysis is immediately followed by 15 seconds of SF5+ sputtering. For depth profiles of the surface and subsurface regions only, the sputtering time was decreased to 1 second for the 5% active agent sample and 2 seconds for both the 25% and 50% active agent samples.

Temperature-controlled depth profiles are obtained using a variable-temperature stage with Eurotherm Controls temperature controller and IPSG V3.08 software. samples are first placed into the analysis chamber at room temperature. The samples are brought to the desired temperature under ultra high-vacuum conditions and are allowed to stabilize for 1 minute prior to analysis. All depth profiling experiments are performed at - 100C and 25C.

### Atomic Force Microscopy (AFM)

AFM is a high resolution surface characterization technique. AFM is used in the art to provide topographical imaging, in addition when employed in Tapping Mode™ can image material and or chemical properties of the surface. Additionally cross-sectioned samples can be analyzed. The technique can be used under ambient, solution, humidified or temperature controlled conditions. Other modes of operation are well known and can be readily employed here by those skilled in the art.

A substrate having a coating as described herein is obtained. AFM is used to determine the structure of the drug polymer layers. AFM may be employed as described in Ranade et al., "Physical characterization of controlled release of paclitaxel from the TAXUS Express2 drug-eluting stent" J. Biomed. Mater. Res. 71(4):625-634 (2004) incorporated herein in its entirety by reference.

Polymer and drug morphologies, coating composition, at least may be determined using atomic force microscopy (AFM) analysis. A multi-mode AFM (Digital Instruments/Veeco Metrology, Santa Barbara, CA) controlled with Nanoscope IIIa and NanoScope Extender electronics is used. Samples are examined in the dry state using AFM before elution of the drug (e.g. rapamycin). Samples are also examined at select time points through a elution period (e.g. 48 hours) by using an AFM probe-tip and flow-through stage built to permit analysis of wet samples. The wet samples are examined in the presence of the same elution medium used for in-vitro kinetic drug release analysis (e.g. PBS-Tween20, or 10 mM Tris, 0.4 wt.% SDS, pH 7.4). Saturation of the solution is prevented by frequent exchanges of the release medium with several volumes of fresh medium. TappingMode™ AFM imaging may be used to show topography (a real-space projection of the coating surface microstructure) and phase-angle changes of the AFM over the sample area to contrast differences in the materials properties. The AFM topography images can be three-dimensionally rendered to show the surface of a coated stent, which can show holes or voids of the coating which may occur as the polymer is absorbed and the drug is eluted over time, for example.

### Scanning Electron Microscopy (SEM) with Focused Ion Beam (FIB) Milling

Coatings on substrates as described herein, and or produced by methods described herein are visualized using SEM-FIB. Alternatively, a coated coupon could be tested in this method. Focused ion beam FIB is a tool that allows precise site-specific sectioning, milling and depositing of materials. FIB can be used in conjunction with SEM, at ambient or cryo conditions, to produce in-situ sectioning followed by high-resolution imaging . FIB -SEM can produce a cross-sectional image of the polymer and drug layers on the substrate. The image can be used to quantitate the thickness of the layers and uniformity of the layer thickness at manufacture and at time points after stenting (or after in-vitro elution at various time points).

A FEI Dual Beam Strata 235 FIB/SEM system is a combination of a finely focused Ga ion beam (FIB) accelerated by 30 kV with a field emission electron beam in a scanning electron microscope instrument and is used for imaging and sectioning the stents. Both beams focus at the same point of the sample with a probe diameter less than 10nm. The FIB can also produce thinned down sections for TEM analysis.

To prevent damaging the surface of the substrate with incident ions, a Pt coating is first deposited via electron beam assisted deposition and ion beam deposition prior to FIB sectioning. For FIB sectioning, the Ga ion beam is accelerated to 30 kV and the sectioning process is about 2 h in duration. Completion of the FIB sectioning allows one to observe and quantify by SEM the thickness of the polymer layers that are, for example, left on the substrate as they are absorbed.

### EXAMPLE 11: DETERMINATION OF THE MICROSTRUCTURE OF A COATING ON A MEDICAL DEVICE

### Atomic Force Microscopy (AFM)

AFM is a high resolution surface characterization technique. AFM is used in the art to provide topographical imaging, in addition when employed in Tapping Mode™ can image material and or chemical properties of the surface. Additionally cross-sectioned samples can be analyzed. The technique can be used under ambient, solution, humidified or temperature controlled conditions. Other modes of operation are well known and can be readily employed here by those skilled in the art.

A device as described herein is obtained. AFM is used to determine the microstructure of the coating. A stent as described herein is obtained. AFM may be employed as described in Ranade et al., "Physical characterization of controlled release of paclitaxel from the TAXUS Express2 drug-eluting stent" J. Biomed. Mater. Res. 71(4):625-634 (2004) incorporated herein in its entirety by reference.

For example, polymer and drug morphologies, coating composition, and physical structure may be determined using atomic force microscopy (AFM) analysis. A multi-mode AFM (Digital Instruments/Veeco Metrology, Santa Barbara, CA) controlled with Nanoscope IIIa and NanoScope Extender electronics is used. Samples are examined in the dry state using AFM before elution of the drug (e.g. rapamycin). Samples are also examined at select time points through a elution period (e.g. 48 hours) by using an AFM probe-tip and flow-through stage built to permit analysis of wet samples. The wet samples are examined in the presence of the same elution medium used for in-vitro kinetic drug release analysis (e.g. PBS-Tween20, or 10 mM Tris, 0.4 wt.% SDS, pH 7.4). Saturation of the solution is prevented by frequent exchanges of the release medium with severl volumes of fresh medium. TappingMode™ AFM imaging may be used to show topography (a real-space projection of the coating surface microstructure) and phase-angle changes of the AFM over the sample area to contrast differences in the materials properties. The AFM topography images can be three-dimensionally rendered to show the surface of a coated stent, which can show holes or voids of the coating which may occur as the polymer is absorbed and the drug is released from the polymer over time, for example.

### Nano X-Ray Computer Tomography

Another technique that may be used to view the physical structure of a device in 3-D is Nano X-Ray Computer Tomography (e.g. such as made by SkyScan), which could be used in an elution test and/or bioabsorbability test, as described herein to show the physical structure of the coating remaining on substrates at each time point, as compared to a scan prior to elution/ bioabsorbtion.

### EXAMPLE 12: DETERMINATION OF THE TOTAL CONTENT OF THE ACTIVE AGENT (AND/OR THE CONTENT OF ACTIVE AGENT REMAINING ON A DEVICE FOLLOWING AN INTERVENTION)

Determination of the total content of the active agent in a coated substrate may be tested using techniques described herein as well as other techniques obvious to one of skill in the art, for example using GPC and HPLC techniques to extract the drug from the coated substrate and determine the total content of drug in the sample.

UV-VIS can be used to quantitatively determine the mass of rapamycin (or another active agent) coated onto the substrates. A UV-Vis spectrum of Rapamycin can be shown and a Rapamycin calibration curve can be obtained, (e.g. λ @ 277nm in ethanol). Rapamycin is then dissolved from the coated substrate in ethanol, and the drug concentration and mass calculated.

In one test, the total amount of rapamycin (or another active agent) present in units of micrograms per substrate is determined by reverse phase high performance liquid chromatography with UV detection (RP-HPLC-UV). The analysis is performed with modifications of literature-based HPLC methods for rapamycin (or the other active agent) that would be obvious to a person of skill in the art. The average drug content of samples (n=10) from devices comprising stents and coatings as described herein, and/or methods described herein are tested.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A method for the preparation of a composition comprising poly(D,L-lactic-co-glycolic acid) containing less than 1.0 wt.-% of acidic impurities, said method comprising:
(I) contacting poly(D,L-lactic-co-glycolic acid) containing acidic impurities, which form salts when being contacted with a solid base, with a solid base, wherein said poly(D,L-lactic-co-glycolic acid) is in either
(a) a solvent-free solution phase, at or above ambient temperature,
(b) a solution phase, at normal atmospheric pressure, in an organic solvent that does not chemically react with the solid base,
(c) a solution phase in a suitable solvent for the creation of a supercritical fluid state, which is a compressed fluid under conditions wherein the temperature is at least 80% of the critical temperature of the fluid and the pressure is at least 50% of the critical pressure of the fluid, or
(d) a solid phase;
forming a salt of the acidic impurity; and
separating the poly(D,L-lactic-co-glycolic acid) from the salt of the acidic impurity; or
(II) dissolving the poly(D,L-lactic-co-glycolic acid) containing acidic impurities in an inert solvent;
contacting poly(D,L-lactic-co-glycolic acid) solution with a metal hydride;
forming a metal salt of the acidic impurity; and
separating the poly(D,L-lactic-co-glycolic acid) from the metal salt of the acidic impurity.

2. The method of claim 1, wherein the method is performed in a supercritical state.

3. The method of claim 1 or 2, wherein the inert solvent is at least one of an organic solvent and a fluorocarbon.

4. The method of claim 1(I), wherein the solid base is selected from the group consisting of: MgO, LiH, LiAlH₄, NaH, NaBH₄, KH, MgH₂, and CaH₂.

## Patentansprüche

1. Verfahren zur Herstellung einer Zusammensetzung, die Poly(D,L-lactid-*co-*glycolid) umfasst, die weniger als 1,0 Gew.-% saure Verunreinigungen enthält, wobei das Verfahren umfasst:
(I) Inkontaktbringen von Poly(D,L-lactid-*co*-glycolid), das saure Verunreinigungen enthält, die Salze bilden, wenn sie mit einer festen Base in Kontakt gebracht werden, mit einer festen Base, wobei das Poly(D,L-lactid-*co*-glycolid) entweder in
(a) einer lösungsmittelfreien Lösungsphase bei oder oberhalb der Umgebungstemperatur,
(b) einer Lösungsphase bei normalen Atmosphärendruck in einem organischen Lösungsmittel, das nicht chemisch mit der festen Base reagiert,
(c) einer Lösungsphase in einem geeigneten Lösungsmittel für die Erschaffung eines überkritischen flüssigen Zustands, was eine komprimierte Flüssigkeit unter Bedingungen ist, wobei die Temperatur mindestens 80% der kritischen Temperatur der Flüssigkeit beträgt, und der Druck mindestens 50% des kritischen Drucks der Flüssigkeit beträgt, oder
(d) einer Festphase vorliegt;
Bilden eines Salzes der sauren Verunreinigung; und
Trennen des Poly(D,L-lactid-*co*-glycolids) vom Salz der sauren Verunreinigung; oder
(II) Lösen des Poly(D,L-lactid-*co*-glycolids), das saure Verunreinigungen enthält, in einem inerten Lösungsmittel;
Inkontaktbringen der Poly(D,L-lactid-*co*-glycolid)-Lösung mit einem Metallhydrid;
Bilden eines Metallsalzes der sauren Verunreinigung; und
Trennen des Poly(D,L-lactid-*co*-glycolids) vom Metallsalz der sauren Verunreinigung.

2. Verfahren nach Anspruch 1, wobei das Verfahren in einem überkritischen Zustand durchgeführt wird.

3. Verfahren nach Anspruch 1 oder 2, wobei das inerte Lösungsmittel mindestens eines von einem organischen Lösungsmittel und einem Fluorcarbon ist.

4. Verfahren nach Anspruch 1 (I), wobei die feste Base aus der Gruppe bestehend aus: MgO, LiH, LiAlH₄, NaH, NaBH₄, KH, MgH₂ und CaH₂ ausgewählt ist.

## Revendications

1. Procédé pour la préparation d'une composition comprenant du poly(acide D,L-lactique-co-glycolique) contenant moins de 1,0 % en poids d'impuretés acides, ledit procédé comprenant :
(I) la mise en contact du poly(acide D,L-lactique-co-glycolique) contenant des impuretés acides, qui forment des sels lorsqu'elles sont mises en contact avec une base solide, avec une base solide, dans lequel ledit poly(acide D,L-lactique-co-glycolique) est dans soit
(a) une phase de solution exempte de solvant, à ou au-dessus de la température ambiante,
(b) une phase de solution, à la pression atmosphérique normale, dans un solvant organique qui ne réagit pas chimiquement avec la base solide,
(c) une phase de solution dans un solvant approprié pour la création d'un état de fluide supercritique, qui est un fluide comprimé dans des conditions dans lesquelles la température est au moins 80 % de la température critique du fluide et la pression est au moins 50 % de la pression critique du fluide, ou
(d) une phase solide ;
la formation d'un sel de l'impureté acide ; et
la séparation du poly(acide D,L-lactique-co-glycolique) du sel de l'impureté acide ; ou
(II) la dissolution du poly(acide D,L-lactique-co-glycolique) contenant des impuretés acides dans un solvant inerte ;
la mise en contact de la solution de poly(acide D,L-lactique-co-glycolique) avec un hydrure métallique ;
la formation d'un sel métallique de l'impureté acide ; et
la séparation du poly(acide D,L-lactique-co-glycolique) du sel métallique de l'impureté acide.

2. Procédé selon la revendication 1, dans lequel le procédé est réalisé dans un état supercritique.

3. Procédé selon la revendication 1 ou 2, dans lequel le solvant inerte est au moins un solvant parmi un solvant organique et un fluorocarbone.

4. Procédé selon la revendication 1(I), dans lequel la base solide est choisie dans le groupe constituée par : le MgO, le LiH, le LiAlH4, le NaH, le NaBH4, le KH, le MgH₂ et le CaH2.
